# EUROPÄISCHE PATENTANMELDUNG

(11) **EP 4 006 796 A1**
(43) Veröffentlichungstag der Anmeldung: **01.06.2022**
(21) Anmeldenummer: 20210115.0
(22) Anmeldetag: 26.11.2020
(51) Int. Cl.: G06Q 10/06, G06Q 50/26, G16H 50/80

(54) **ELEKTRONISCHES SYSTEM ZUM TRACING VON NUTZERN, INSBESONDERE ZUR BEKÄMPFUNG EINER PANDEMIE**

(71) Anmelder: culture4life GmbH, 10117 Berlin (DE)
(72) Erfinder: HENNIG, Patrick, 10117 Berlin (DE)
(74) Vertreter: Richardt Patentanwälte PartG mbB

(57) **Zusammenfassung**

Die Erfindung betrifft ein elektronisches System (200) mit Nutzergeräten (204), einem Server (202) zum Tracing von Nutzern, die sich temporär in verschiedenen räumlichen Bereichen (Venues) aufhalten, für eine Entität (246), wobei das elektronische System zur Durchführung der folgenden Schritte konfiguriert ist:
- anlässlich einer Registrierung: Speicherung von Nutzerdaten (300) auf dem Server (202), wobei die Nutzerdaten mit einem für den jeweiligen Nutzer individuellen U-ser-Data-Key (310) verschlüsselt sind; und Speicherung der User-IDs und eines Nutzer-individuellen zur Entschlüsselung der verschlüsselten Nutzerdaten erforderlichen User-Data-Entschlüsselungsschlüssels (310) jeweils in demjenigen der Nutzergeräte, welches dem Nutzer zugeordnet ist;
- Im Zuge eines Check-ins eines der Nutzer gegenüber einem der Venues, Erzeugung einer Trace-ID, die repräsentativ ist für eine Kombination aus User-ID und einer aktuellen Zeitinformation, und Speicherung der Trace-ID verknüpft mit der Venue-ID des einen Venue auf dem Server (202),
- Empfang einer Mitteilung beinhaltend die User-ID eines der Nutzer;
- Erzeugung von Kandidaten-Trace-IDs für den in der Mitteilung identifizierten Nutzer, wobei die Kandidaten-Trace-IDs mögliche zurückliegende Trace-IDs sind, die innerhalb eines vorgegebenen vergangenen Zeitraums liegen, und
- Verwendung der erzeugten Kandidaten-Trace-IDs als Suchkriterium durch den Server (202), um entsprechende auf dem Server für den vergangenen Zeitraum gespeicherte Trace-IDs aufzufinden, um die Venue-IDs der vom Nutzer besuchten Venues zu ermitteln,
- Ausgabe der ermittelten Venue-IDs.

## Beschreibung

### Technisches Gebiet

Die Erfindung betrifft ein elektronisches System zum Tracing von Nutzern, insbesondere zum Tracing von Nutzern in Notfallszenarien wie z.B. bei der Bekämpfung einer Pandemie, wie zum Beispiel Covid-19, sowie ein entsprechendes digitales Speichermedium, auf dem ein Computerprogramm bzw. eine App gespeichert ist.

### Stand der Technik

Zur Begrenzung der Verbreitung von Covid-19 wird bislang so vorgegangen, dass bei Besuch einer öffentlichen Veranstaltung oder einer Gaststätte die betreffende Person ihre persönlichen Daten angeben muss. Die damit einhergehenden Dokumentationspflichten sind für die entsprechenden Veranstalter bzw. Betreiber von Restaurants aufwendig, zumal auch die datenschutzrechtlichen Bestimmungen eingehalten werden müssen.

Außerdem kann mit dem bisherigen Verfahren die Sicherheit der sensiblen, personenbezogenen Daten der Besucher wie z.B. Anschrift und Telefonnummer, nicht in dem Maße gewährt werden, die wünschenswert wäre, da die Betreiber von Restaurants und Kulturveranstaltungsräumen nicht darauf vorbereitet sind, sensible Daten einer Vielzahl von Personen sicher zu speichern. Außerdem ist bereits der Umstand, dass die Adressdaten der Besucher den Betreibern der Veranstaltungslokalitäten offengelegt werden müssen, ein Sicherheitsrisiko, da hierdurch der Betreiber der Lokalität zumindest bezüglich seiner eigenen Räumlichkeiten ein Tracing von Nutzern durchführen kann und der darüber hinaus die Identität der Gäste kennt. Demgegenüber werden erfindungsgemäß ein computerimplementiertes Verfahren, ein elektronisches System sowie einzelne Systemkomponenten wie beansprucht geschaffen, welches ein weitgehend automatisiertes Tracing unter Wahrung von datenschutzrechtlichen Vorschriften gewährleistet.

### Technisches Problem und grundlegende Lösungen

Vor diesem Hintergrund besteht ein Bedarf an verbesserten Verfahren zum Tracing von Besuchern von Veranstaltungslokalitäten und anderen räumlichen Bereichen insofern, dass die vorangehend erwähnten Nachteile damit zumindest teilweise vermeidbar sind.

Der Erfindung liegt demgegenüber die Aufgabe zugrunde, ein verbessertes Verfahren und System zum Tracing von Besuchern zu schaffen, welches einen besseren Schutz sensibler personenbezogener Daten bietet.

Die der Erfindung zugrunde liegenden Aufgaben werden jeweils mit den Merkmalen der unabhängigen Patentansprüche gelöst. Ausführungsformen der Erfindung sind in den abhängigen Ansprüchen angegeben. Die im Folgenden aufgeführten Ausführungsformen sind frei miteinander kombinierbar, sofern sie sich nicht gegenseitig ausschließen.

In einem Aspekt betrifft die Erfindung ein elektronisches System mit Nutzergeräten, die jeweils einem Nutzer zugeordnet sind und mit zumindest einem Server zum Tracing von Nutzern, die sich temporär in verschiedenen räumlichen Bereichen (Venues) aufhalten. Das Tracing der Nutzer wird für eine Entität vorgenommen. Das elektronische System ist zur Durchführung der folgenden Schritte konfiguriert:
- anlässlich einer Registrierung: Speicherung von Nutzerdaten der Nutzer auf dem Server, wobei die Nutzerdaten mit einem für den jeweiligen Nutzer individuellen User-Data-Key (also einem symmetrischen oder asymmetrischen öffentlichen Verschlüsselungsschlüssel für die Verschlüsselung der Nutzerdaten) verschlüsselt sind, wobei insbesondere eine User-ID des betreffenden Nutzers verknüpft mit den verschlüsselten Nutzerdaten auf dem Server gespeichert werden; und Speicherung der User-IDs und eines Nutzer-individuellen zur Entschlüsselung zur Entschlüsselung der verschlüsselten Nutzerdaten jeweils in demjenigen der Nutzergeräte, welches dem Nutzer zugeordnet ist, wobei vorzugsweise der User-Data-Key und/oder der User-Data-Entschlüsselungsschlüssel (der User-Data-Key und der User-Data Entschlüsselungsschlüssel können identische symmetrische Schlüssel sein) geschützt in dem Nutzergerät des jeweiligen Nutzer gespeichert wird und dem Server nicht zugänglich ist;
- Im Zuge eines Check-ins eines der Nutzer gegenüber einem der Venues, Erzeugung einer Trace-ID durch das Nutzergerät des einen Nutzers, wobei die Trace-ID repräsentativ ist für eine Kombination aus User-ID des einen Nutzers und einer aktuellen Zeitinformation, und Speicherung der Trace-ID verknüpft mit der Venue-ID des einen Venue auf dem Server, wobei die Trace-ID als Zugriffsschlüssel gespeichert wird,
- Empfang einer Mitteilung beinhaltend die User-ID eines der Nutzer bezüglich welchem das Tracing durchgeführt werden soll; Beispielsweise kann die Mitteilung von einem Entitäts-Gerät der Entität gesendet und von dem Server empfangen werden; In einem anderen Beispiel kann die Mitteilung auch von einem Entitäts-Gerät der Entität gesendet, von dem Nutzergerät empfangen und an den Server weitergeleitet werden;

- Erzeugung von Kandidaten-Trace-IDs für den in der Mitteilung identifizierten Nutzer, wobei die Kandidaten-Trace-IDs mögliche zurückliegende Trace-IDs sind, die innerhalb eines vorgegebenen vergangenen Zeitraums liegen, und
- Verwendung der erzeugten Kandidaten-Trace-IDs als Suchkriterium durch den Server, um entsprechende auf dem Server für den vergangenen Zeitraum gespeicherte Trace-IDs aufzufinden, so dass die Venue-IDs derjenigen Venues gegenüber welchen der Nutzer während des vergangenen Zeitraums einen Check-in durchgeführt hat, ermittelt werden,
- Ausgabe der Venue-IDs der ermittelten Venues zur Identifizierung derjenigen Venues, die der Nutzer während des vergangenen Zeitraums besucht hat, durch den Server.

Dies kann vorteilhaft sein, da auf dem Server zwar die Nutzerdaten der Nutzer sowie die im Zuge von Check-in Ereignissen bei verschiedenen Venues die Venue-IDs verknüpft mit den Trace-IDs der verschiedenen Nutzer gespeichert sind, hierbei aber sichergestellt ist, dass weder der Betreiber des Venue noch der Betreiber des Servers ohne Autorisierung durch den Nutzer auf die auf dem Server gespeicherten Nutzerdaten zugreifen können, da für die Entschlüsselung der Nutzerdaten nötigen Entschlüsselungsschlüssel zum einen nutzerindividuell erstellt wurden und weil zum anderen diese Entschlüsselungsschlüssel geschützt in dem Nutzergerät gespeichert sind. Das bedeutet, ein Bekanntwerden des Nutzerdaten-Entschlüsselungsschlüssels eines Nutzers hat keine Auswirkungen auf die Sicherheit der Nutzerdaten der anderen Nutzer. Und die Identitäten der beim Server registrierten Nutzer sind zwar sicher und zentral auf dem Server hinterlegt zusammen mit den Check-in Datensätzen dieser Nutzer. Da die Check-in Datensätze, insbesondere die verschlüsselten Ausweisdaten des Nutzers, jedoch mit dem Entitätenschlüssel (und optional auch mit einem Venue-Schlüssel) verschlüsselt sind, kann weder der Server noch der Venue erkennen, welche Person den Venue besucht hat. Falls der Venue vor dem Hochladen der Check-in Datensätze diese mit seinem Venue-Schlüssel verschlüsselt, kann der Server gemäß Ausführungsformen noch nicht einmal feststellen, wie viele Besucher ein Venue an einem bestimmten Zeitpunkt hatte (falls die Zeitstempel mitverschlüsselt werden).

Ausführungsformen der Erfindung können somit den Vorteil haben, dass eine anonyme und sichere Übertragung und Speicherung von Kontaktinformationen ermöglicht wird, die ein Tracing einzelner Nutzer erlaubt, allerdings nur, wenn der Nutzer dem Tracing auch zustimmt. Besucher und Gäste von öffentlichen oder privaten Veranstaltungen werden dadurch in die Lage versetzt, dem Veranstalter anonym ihre Kontaktdaten mitzuteilen. Nur im Falle einer Infektion wird den örtlichen Gesundheitsbehörden mit Zustimmung des Gastes und vorzugsweise auch mit Zustimmung des Veranstalters der Zugriff auf die Kontaktdaten ermöglicht. Die Kontaktdaten können genutzt werden, um mögliche Ansprechpartner zu informieren.

Beispielsweise kann das System dazu verwendet werden, die Kontaktverfolgung für Veranstaltungsorte, Restaurants und andere räumliche Bereiche (Zugabteil, Flugzeug, Stadien, Konzerthallen, Gebäude, Räume, Konzertflächen im Freien etc.) sicherer und einfacher zu gestalten und insbesondere eine sichere und maschinelle und damit effiziente und skalierbare Tracing-Lösung bereitzustellen.

Das anonyme und sichere Tracing-System bzw. Verfahren kann in verschiedenen Anwendungsszenarien eingesetzt werden. Ein Anwendungsbeispiel ist das Tracing von Infizierten im Falle einer Pandemie wie z.B. Covid-19 oder anderer ansteckender Krankheiten. Ein anderes Anwendungsbeispiel ist die Nachverfolgung von Personen im Falle anderer Arten von Notfällen oder zur Aufklärung von Sicherheitsvorfällen. Beispielsweise könnte ein Besitzer eines räumlichen Bereiches mit hohem Besucherverkehr wie z.B. Zoos, Freizeitparks oder Bahnhöfen das System nutzen, um Besucher beim Betreten des räumlichen Bereiches im Zuge eines Check-ins am Eingang anonym zu registrieren. Das bedeutet, dass der Besitzer zwar die Check-in Daten (die Venue-ID verknüpft mit der Trace-ID der Nutzer und ggf. weiteren Daten) auf dem Server speichert, ohne dass der Besucher hierfür dem Besitzer oder Betreiber des Venue seine Identität offenlegen müsste. Falls jedoch auf dem Venue sich ein Sicherheitsvorfall ereignen sollte, z.B. Besucher verletzt worden sein sollten, kann der Betreiber der Venue in einem öffentlichen Aufruf darum bitten, sich bei der zuständigen Entität (z.B. zuständige Behörde oder kompetente, vertrauenswürdige Organisation.) zu melden, um z.B. als Zeuge mitzuwirken (deren Anwesenheit durch Check-in und optional auch Check-Out Ereignisse und serverseitig hinterlegte Datensätze dokumentiert ist.

Nach Ausführungsformen sind die user-IDs Datenwerte, die nutzer-individuell erstellt werden, und die die Identität der Nutzer (insbesondere Name, Kontaktdaten, und/oder Adresse sowie persönliche Attribute wie Kontonummer, Geburtstag, Geburtsort etc.) nicht offenbaren.

Nach Ausführungsformen enthält die Trace-ID die User-ID nur in kodierter oder verschlüsselter Form, sodass der Server anhand der Trace-ID nicht die User-ID des zugehörigen Nutzers erkennen kann.

Nach Ausführungsformen sind die Trace-IDs Datenwerte, die die Identität der Nutzer (insbesondere Name, Kontaktdaten, und/oder Adresse sowie persönliche Attribute wie Kontonummer, Geburtstag, Geburtsort etc.) nicht offenbaren.

Nach Ausführungsformen der Erfindung wird die im Zuge des Check-ins erzeugte Trace-ID mittels einer Ableitfunktion berechnet, nämlich als Funktion der Kombination aus der zu repräsentierenden User-ID und der zu repräsentierenden aktuellen Zeitinformation beim Check-in. Jede der Kandidaten-Trace-IDs wird jeweils mittels der gleichen Ableitfunktion als Funktion der Kombination der in der Mitteilung enthaltenen User-ID und einer der Kandidaten-Zeitinformationen für einen möglichen in der Vergangenheit liegenden Check-in-Zeitpunkt, berechnet. Die Ableitungsfunktion umfasst vorzugsweise eine Rundung der aktuellen bzw. Kandidaten-Zeitinformation auf Zeitpunkte innerhalb vordefinierter Zeitintervalle, wobei die Zeitintervalle insbesondere Minuten oder Stunden oder Tage sind. Beispielsweise kann im Zuge des Check-ins die aktuelle Zeit vom Nutzergerät kontinuierlich erfasst werden, nach der Erfassung auf die volle Minute abgerundet werden, und dann zusammen mit der User-ID als Input an die Ableitfunktion übergeben werden. Die Tracing-ID und die Venue-ID kann sodann mit optionalen weiteren Daten verknüpft und in Form eines QR-Codes kodiert und auf dem Nutzergerät zum Zwecke des Check-ins angezeigt werden. Das Nutzergerät ist dazu konfiguriert, automatisch eine neue Trace-ID und ggf. auch einen neuen QR-Code zu erzeugen, wenn das Zeitintervall (hier z.B. Minute) verstrichen ist und eine aktuellere Zeitinformation (aktuelle Minute, abgerundet) erhalten wurde. Wenn der alte QR-Code bis dahin nicht für den Check-in verwendet wurde, wird die darin enthaltene Trace-ID auch nicht auf dem Server gespeichert und wird vom Nutzergerät verworfen.

Dies kann vorteilhaft sein, da die Trace-ID zeitabhängig gebildet wird und es somit unmöglich ist, die Trace-IDs einzelnen Nutzern zuzuweisen oder auch nur nach Nutzer zu gruppieren. Der Betreiber des Venues und des Servers können also auf Basis der so gebildeten Trace-IDs noch nicht einmal anonyme Bewegungsprofile der einzelnen Besucher erstellen, da die Trace-IDs als Ableitung eines konstanten Datenwerts (User-ID) und eines sich regelmäßig ändernden Zeitwertes gebildet werden. Dies erhöht die Sicherheit der Nutzer vor unberechtigtem Tracing, sollte ein Angreifer sich Zugriff auf das Venue-Gerät, gegenüber welchem das Check-in vorgenommen wird und/oder Zugriff auf den Server verschaffen.

Nach Ausführungsformen der Erfindung ist das Nutzergerät dazu konfiguriert, im Zuge der Registrierung gegenüber dem Server einen Zufallswert ("userTracingSecret") zu erzeugen und zugriffsgeschützt ausschließlich im Nutzergerät zu speichern. Die im Zuge des Check-ins und/oder der Rückverfolgung eines Nutzers zur Erzeugung der Kandidaten-Trace-IDs verwendete Ableitfunktion berechnet die Trace-ID als Funktion (auch) des Zufallswerts ("userTracingSecret"). Das Nutzergerät ist dazu konfiguriert, von dem Benutzer, dem es zugewiesen ist, eine Bestätigung zur Übermittlung des Zufallswerts einzuholen, und erst in Antwort auf die Bestätigung den Zufallswert an den Server zu übermitteln (z.B. innerhalb eines Tracinggeheimnis-Transferobjekts) um diesem die Ableitung der Kandidaten-Trace-IDs zu ermöglichen. Zusätzlich oder alternativ dazu kann das Nutzergerät dazu konfiguriert sein, erst in Antwort auf die Bestätigung den Zufallswert an ein Entitäten-Gerät der Entität zu übermitteln (z.B. innerhalb eines Tracinggeheimnis-Transferobjekt) um der Entität die Ableitung der Kandidaten-Trace-IDs zu ermöglichen.

Je nach Ausführungsformen haben die auf dem Nutzergerät, dem Server und/oder dem Entitäten-Gerät installierten Anwendungsprogramme bzw. Apps die erforderlichen Schnittstellen, um direkt oder indirekt miteinander über ein Netzwerk Daten auszutauschen.

Dies kann vorteilhaft sein, dass ein Angreifer, der die User-ID errät und ggf. sogar die Ableitfunktion in Erfahrung bringt, dennoch nicht die auf dem Server gespeicherten Trace-IDs manipulieren kann dadurch, dass er die Trace-IDs eines Nutzers innerhalb eines bestimmten Zeitpunkt berechnen und manipulieren (löschen und/oder ersetzen oder durch "erfundene" Trace-IDs ergänzen) kann. Der Angreifer müsste nämlich zusätzlich auch in das Nutzergerät des Nutzers eindringen und den dort bei der Registrierung erzeugten und geschützt gespeicherten Zufallswerts ("userTracingSecret") in Erfahrung bringen, um für diesen NutzerTrace-IDs berechnen und damit erkennen, erzeugen und manipulieren zu können.

Nach Ausführungsformen ist das Nutzergerät dazu konfiguriert, im Zuge der Registrierung gegenüber dem Server einen Zufallswert zu erzeugen, im Folgenden als userVerificationSecret bezeichnet, und zugriffsgeschützt ausschließlich im Nutzergerät zu speichern. Das Nutzergerät ist dazu konfiguriert, im Zuge des Check-ins gegenüber dem Venue das userVerificationSecret zu verwenden, um mit diesem einen Verifikationsdatenwert als Funktion der Trace-ID und der verschlüsselten Ausweis-Daten zu berechnen und verknüpft mit der Trace-ID auf dem Server zu speichern. Die Speicherung auf dem Server kann je nach Ausführungsform direkt erfolgen oder indirekt dadurch, dass die Check-in Daten zunächst von einem Venue-Gerät des Venues erfasst werden und das Venue-Gerät diese Daten dann an den Server weiterleitet, um sie serverseitig zu speichern.

Gemäß Ausführungsformen der Erfindung ist der Entität ein asymmetrisches kryptographisches Schlüsselpaar mit einem öffentlichen und einem privaten Entitätenschlüssel zugeordnet. Das Nutzergerät des Nutzers ist dazu konfiguriert, im Zuge der Registrierung die bereits mit dem Nutzerdaten-Schlüssel verschlüsselten Nutzerdaten zusätzlich mit dem öffentlichen Entitätenschlüssel zu verschlüsseln.

Zusätzlich oder alternativ dazu ist das Nutzergerät des Nutzers dazu konfiguriert, im Zuge des Check-ins den für die Entschlüsselung dieser verschlüsselten Nutzerdaten erforderlichen Nutzerdaten-Entschlüsselungsschlüssel durch den öffentlichen Entitätenschlüssel zusammen mit der User-ID des Nutzers zu verschlüsseln, um verschlüsselte Ausweis-Daten zu erzeugen. Im Zuge des Check-ins speichert das Nutzergerät die verschlüsselten Ausweis-Daten verknüpft mit der Venue-ID des einen Venue auf dem Server. Diese Speicherung kann direkt erfolgen, wenn z.B. der Venue-Betreiber kein Venue-Gerät besitzt und das Nutzergerät den "Check-in" selbst vornimmt, indem es die Daten des Venues erfasst und zum Check-in verwendet und die Check-in Daten samt Venue-ID an den Server zur Speicherung übermittelt. Die Speicherung kann aber auch indirekt mittels des Venue-Geräts erfolgen, indem das Venue-Gerät die Check-in Daten des Nutzers erfasst, mit der Venue-ID des Venues, dem das Venue-Gerät zugeordnet ist, verknüpft, und zur Speicherung an den Server übermittelt.

Die Verwendung eines Entitäten-schlüssels, z.B. eines öffentlichen Verschlüsselungsschlüssels einer Behörde, z.B. des Gesundheitsamts zu den genannten Zwecken kann vorteilhaft sein, da das Amt, wenn es Zugriff auf den Server erhält, mit seinem zum öffentlichen Entitätenschlüssel korrespondierenden privaten Entitätenschlüssel die Ausweisdaten, die verknüpft mit der Trace-ID gespeichert sind, zu entschlüsseln, und hierdurch die User-ID und den zugehörigen Nutzerdaten-Entschlüsselungsschlüssel zu erhalten. Mit dem Nutzerdaten-Entschlüsselungsschlüssel wiederum kann das Amt die verschlüsselten Nutzerdaten entschlüsseln, um die Identität des Nutzers zu erfahren.

Außerdem können diese Merkmale den Vorteil haben, dass die Tracing-Datensätze auf dem Server weniger Speicherplatz verbrauchen und die Tracing-Daten in einem graphischen Code wie z.B. einem QR-Code kodiert werden können: da die Nutzer-ID und der User-Data-Entschlüsselungsschlüssel (Nutzerdaten-Entschlüsselungsschlüssel) zur Erzeugung der verschlüsselten Ausweisdaten verschlüsselt werden, nicht aber die kompletten Nutzerdaten, die auch recht umfangreich sein können, können diese beim Check-in übertragenen Daten problemlos in einem QR Code kodiert werden, was einen automatische Check-in erleichtert.

Nach Ausführungsformen umfasst das elektronische System zumindest ein Entitäts-Gerät, das der Entität zugeordnet ist. Das Entitäts-Gerät ist dazu konfiguriert, nacheinander mehrere asymmetrische kryptographische Schlüsselpaare zu erzeugen, die der Entität zugewiesen sind, wobei bei Erzeugung eines neuen Entitäten-Schlüsselpaares das bisher gültige invalidiert wird, und wobei jedem der Entitäten-Schlüsselpaare eine Schlüsselversionsnummer zugewiesen ist.

Das zumindest eine Entitäts-Gerät ist dazu konfiguriert, den öffentlichen Schlüssel des erzeugten und aktuell gültigen Schlüsselpaares an die Nutzergeräte zu übertragen. Die Nutzergeräte sind dazu konfiguriert, immer nur den jüngsten übertragenen Entitätenschlüssel zur Erzeugung der mehrfach verschlüsselten Nutzerdaten und/oder zur Erzeugung der verschlüsselten Ausweis-Daten zu verwenden.

Bei den Entitäts-Geräten kann es sich z.B. um Datenverarbeitungsgeräte, z.B. Desktop-Computer oder Notebooks, oder sonstige Computer insb. von Mitarbeitern einer Behörde handeln oder um Datenverarbeitungsgeräte von Drittanbietern, die von der Entität zur Erfüllung einer Aufgabe der Entität genutzt wird.

Beispielsweise kann die Erzeugung eines neuen Entitäten-Schlüsselpaares täglich erfolgen, z.B. in Antwort auf das erstmalige Starten einer Entitäten-Tracing-Applikation auf einem der Entitäten-Geräte der Entität durch einen der Mitarbeiter, oder an einem vordefinierten Zeitpunkt (z.B. täglich um 7:00 Uhr morgens an einem vordefinierten Entitäten-Gerät).

Beispielsweise kann die Versionsnummer des jeweils verwendeten Entitäten-schlüssels verknüpft mit den mehrfach verschlüsselten Nutzerdaten und/oder verknüpft mit den verschlüsselten Ausweis-Daten auf dem Server gespeichert werden, sodass das Entitäten-Gerät anhand der Versionsnummer den zur Entschlüsselung geeigneten privaten Entitätenschlüssel mit der gleichen Versionsnummer identifizieren kann.

Die regelmäßige Ausstellung neuer Entitätenschlüsselpaare erhöht die Sicherheit, da ein Angreifer, der einen privaten Entitäten-Entschlüsselungsschlüssel in Erfahrung gebracht haben sollte, damit die allermeisten Check-in bzw. Ausweisdaten auf dem Server nicht entschlüsseln könnte, da diese mit einem anderen (tagesaktuellen) Entitätenschlüssel verschlüsselt sind. Ohne Kenntnis der Schlüssel-ID wüsste der Angreifer auch nicht, welche Check-in Datensätze er mit dem in Erfahrung gebrachten Entschlüsselungsschlüssel entschlüsseln könnte.

Nach Ausführungsformen der Erfindung besteht das zumindest ein Entitäts-Gerät aus einer Vielzahl von Entitätsgeräten. Dasjenige der Entitäts-Geräte, welches das aktuelle asymmetrische Entitäten-Schlüsselpaar erzeugt, sendet den privaten Schlüssel des aktuell erzeugten asymmetrischen Entitäten-Schlüsselpaares in verschlüsselter Form an alle anderen der Entitäts-Geräte über ein Netzwerk, z.B. das Internet und/oder ein Intranet. Jedes der Entitäts-Geräte ist dazu konfiguriert, eine Mitteilung eines Notfalls mit Bezug zu einem der Nutzer zu erzeugen und an den Server zu senden. Zusätzlich oder alternativ dazu ist das Entitäts-Gerät dazu konfiguriert, mittels eines der bisher empfangenen privaten Entitätenschlüssel die verschlüsselten Nutzerdaten und/oder die verschlüsselten Ausweis-Daten zu entschlüsseln.

Dies kann vorteilhaft sein, da hierdurch sichergestellt ist, dass verschiedene Mitarbeiter der Entität, die verschiedene Entität-Geräte nutzen, dennoch an jedem Tag den gleichen privaten Entitätenschlüssel zur Entschlüsselung der Daten von Nutzern besitzen, bezüglich welcher ein Notfall (z.B. Positiv-Test im Pandemiefall) vorliegt. Somit wird eine Tracing-Lösung bereitgestellt, die sicher und skalierbar ist, da sie eine dezentrale, mehrere Entität-Geräte umfassende IT-Infrastruktur aufbaut, welche regelmäßig (insb. täglich) neu erzeugte Entitäten-Schlüsselpaare zur Ver- und Entschlüsselung von Nutzerdaten und/oder Ausweis-Daten beruht.

Nach Ausführungsformen der Erfindung umfasst das elektronische System mehrere Venue-Geräte. Jedem der Venues ist ein oder mehrere der Venue-Geräte zugewiesen.

Beispielsweise kann es sich bei dem Venue um ein Lokal mit mehreren Eingängen handeln, wobei an jedem Eingang ein Venue-Gerät für den automatischen Check-in und optional auch für den automatischen Check-out von Gästen angebracht ist. Gemäß einem anderen Beispiel ist der Venue ein Zug oder Zugabteil oder ein Flugzeug oder ein einzelner Sitz eines Flugzeugs und an den Ein- und/oder Ausgängen der jeweiligen Venues ist mindestens ein Venue-Gerät angebracht.

Nach Ausführungsformen der Erfindung führt der Nutzer den Check-in gegenüber einem der Venue-Geräte durch, wobei das eine Venue-Gerät eine Kommunikationsschnittstelle zur direkten Kommunikation mit dem Nutzergerät eines eincheckenden Nutzers beinhaltet. Das Nutzergerät und das eine Venue-Gerät sind so konfiguriert, dass im Zuge des Check-ins Folgendes erfolgt:
- Erzeugung des Check-in Datenpakets durch das Nutzergerät, wobei das Check-in Datenpaket die Trace-ID und vorzugweise auch die verschlüsselten Ausweisdaten kodiert;
- Übertragung des Check-in Datenpakets vom Nutzergerät über die direkte Kommunikationsschnittstelle an das Venue-Gerät; und
- Dekodieren des empfangenen Check-in Datenpakets durch das Venue-Gerät, um die Trace-ID und vorzugsweise auch die Ausweisdaten des eincheckenden Nutzers zu erhalten und diese verknüpft mit der Venue-ID des Venue, dem das Venue-Gerät zugewiesen ist, auf dem Server zu speichern;

Bei der direkten Kommunikationsverbindung kann es sich z.B. um eine Nahfeld-Kommunikationsverbindung oder um einen optischen Datenübertragungskanal handeln. Die Datenübertragung über den optischen Datenübertragungskanal umfasst eine Erfassung eines graphischen Codes, der das Check-in Datenpaket kodiert und auf einer Anzeige des Nutzergeräts angezeigt wird, durch einen optischen Sensor des Venue-Geräts. Das Venue-Gerät dekodiert die in dem Code (z.B. QR-Code oder Barcode) enthaltenen Daten, darunter z.B. die Trace-ID und optional weitere Daten wie z.B. die verschlüsselten Ausweisdaten, Verifikationswerte, QR-Code-Versionsnummer, Zeitstempel des Check-ins und/oder weitere Daten, und speichert diese verknüpft mit der in dem Venue-Gerät hinterlegten Venue-ID auf dem Server. In einer Ausführungsform ist das Venue-Gerät das Smartphone des Betreibers des Venues, auf welchem z.B. eine Venue-App eines Tracing-Dienstanbieters installiert ist, die die Venue-Gerät-seitigen Schritte des Tracingverfahrens implementiert.

Bei der Nahfeld-Kommunikationsverbindung kann es sich z.B. um eine Funkverbindung, eine Infrarot-Verbindung, eine Bluetooth-Verbindung oder andere Verbindungsformen im Nahfeld des Venue-Geräts handeln.

Diese Ausführungsvariante kann vorteilhaft sein, da geeignete Venue-Geräte den Check-in Prozess stark vereinfachen und beschleunigen können und hierbei eine nahtlose Integration der erfassten Check-in Daten in das Tracingsystem ohne Zusatzaufwand für den Besucher oder Venue-Betreiber ermöglicht wird.

Gemäß einer anderen Ausführungsform umfasst das elektronische System zumindest ein Trägerobjekt mit einem graphischen Venue-Code eines der Venues. Der Venue-Code kodiert eine Venue-ID, unter welcher dieser Venue bei dem Server registriert ist. Der Nutzer nimmt den Check-in gegenüber diesem Venue vor, wobei das Nutzergerät so konfiguriert ist, dass im Zuge des Check-ins Folgendes erfolgt:
- Erfassung des graphischen Venue-Codes mit einer Kamera des Nutzergerätes; und
- Dekodierung des Venue-Codes durch das Nutzergerät, um die Venue-ID des Venues zu erhalten.

Die Übertragung der Trace-ID und vorzugsweise auch der Ausweisdaten des eincheckenden Nutzers erfolgt an den Server durch das Nutzergeräts, wobei hierbei auch die dekodierte Venue-ID verknüpft mit der Trace-ID auf dem Server gespeichert wird. Bei dem Venue-Code vorzugsweise um einen QR-Code oder Barcode handelt, der auf einem Aufdruck oder einer Digitalanzeige sichtbar ist, handelt und/oder wobei es sich bei dem Trägerobjekt vorzugsweise handelt um einen Tischaufsteller oder ein Türschild zu einem Fahrzeugwagen, Fahrzeugabteil, Gebäude oder Zimmer handelt.

Dies kann vorteilhaft sein, da der Betreiber des Venues kein eigenes Venue-Gerät vorhalten oder installieren muss, um den Besuchern das sichere Tracingverfahren nach Ausführungsformen der Erfindung zur Verfügung zu stellen. Es ist beispielsweise ausreichend, dass der Betreiber eines Restaurants Tischaufsteller auf den Tischen platziert, wobei auf jedem Tischaufsteller die Venue-ID der Gaststädte und optional weitere Daten (wie z.B. Tischnummer) in Form eines QR-Codes kodiert ist. Ein Gast muss also nur mit der Kamera seines Nutzergerätes den QR-Code auf dem Tischaufsteller erfassen, um den oben beschriebenen "Check-in Prozess" innerhalb des gleichen Nutzergeräts durchzuführen. Beispielsweise können die in dem Venue-Code kodierten Daten vom Nutzergerät verwendet werden, ein simuliertes Venue-Gerät mit der ID und ggf. weiteren Daten des Venues zu "personalisieren" und sodann den Check-in mit diesem simulierten Venue-Gerät durchzuführen. Die Trace-ID wird verknüpft mit der Venue-ID und ggf. weiteren Daten von dem Nutzergerät direkt zum Server zur Speicherung auf dem Server übermittelt.

Nach Ausführungsformen der Erfindung ist das elektronische System so konfiguriert, dass von dem Nutzergerät des einen der Nutzer oder von dem Venue-Gerät eine Check-out Zeit an den Server übertragen wird, wenn der Nutzer den Venue verlässt. Die Check-out Zeit wird der zuvor für diesen Nutzer empfangenen Trace-ID zugeordnet, so dass für den Nutzer der Aufenthaltszeitraum an dem Venue in Form einer Kombination der Check-in Zeit und der Check-Out Zeit gespeichert wird.

Nach Ausführungsformen der Erfindung ist das elektronische System konfiguriert zur:
- Erzeugung eines Tracinggeheimnis-Transferobjekts durch das Nutzergerät, wobei das Tracinggeheimnis-Transferobjekt zumindest die User-ID und den Nutzerdaten-Entschlüsselungsschlüssel des Nutzers, dem das Nutzergerät zugewiesen ist, enthält;
- Verschlüsselung des Tracinggeheimnis-Transferobjekts mit einem öffentlichen Schlüssel der Entität durch das Nutzergerät, wobei es sich bei dem öffentlichen Schlüssel insbesondere um den jüngsten aus einer Reihe nacheinander erzeugter öffentlicher Entitätenschlüssel handelt; beispielsweise kann es sich bei dem Entitätenschlüssel um den öffentlichen Teil des für den aktuellen Tag gültigen Entitäten-Schlüsselpaares handeln;
- Übertragung des verschlüsselten Tracinggeheimnis-Transferobjekts über ein Netzwerk von dem Nutzergerät an den Server;
- In Antwort auf den Erhalt des verschlüsselten Tracinggeheimnis-Transferobjekts, Erzeugen einer TAN und Speicherung der TAN verknüpft mit dem verschlüsselten Tracinggeheimnis-Transferobjekt durch den Server, und Ausgabe der TAN an den Nutzer;
- Empfang der TAN von dem Nutzer durch ein der Entität zugewiesenes Entitäts-Gerät;
- Verwenden der TAN durch das Entitäts-Gerät, um in Interoperation mit dem Server die verschlüsselten Nutzerdaten des Nutzers zu empfangen und zu entschlüsseln.

Vorzugsweise wird die Erzeugung des Tracinggeheimnis-Transferobjekts durch das Nutzergerät ausschließlich durch ein explizites Kommando des Nutzers, dem das Nutzergerät zugewiesen ist, initiiert, beispielsweise dadurch, dass der Nutzer auf einer Tracing-App auf seinem Nutzergerät die Funktion "Identität und Besuchshistorie freigeben" auswählt. Beispielsweise kann der Nutzer dies nach Erhalt eines positiven Befundes für eine Infektionskrankheit nach einer Aufforderung des Gesundheitsamts (z.B. per Mail, Brief oder Telefonanruf) tun.

Dies kann vorteilhaft sein, da ohne die explizite Freigabe durch den Nutzer die Entität die Kandidaten-Trace-IDs nicht erzeugen und somit weder die besuchten Venues noch potenzielle Kontaktpersonen des Nutzers bei diesen Venues identifizieren kann. Wenn der betreffende Nutzer, der getraced werden soll, dem Amt seine Tracinggeheimnisse (also alle Daten, die zur Erzeugung der Kandidaten-Trace-IDs für einen bestimmten Nutzer erforderlich sind) z.B. mittels eines Tracinggeheimnis-Transferobjekts übermittelt, versetzt dies das Amt zwar in die Lage, die Identität der potentiellen Kontakte zu erkennen (deren Nutzerdaten können mit den UserDataKeys dieser Nutzer, die Bestandteil der Check-in Daten der besuchten Venues im Fraglichen Zeitraum sind, vom Amt entschlüsselt werden), nicht jedoch, diese potentiellen Kontaktpersonen zu tracen, da diese hierfür deren Tracinggeheimnis an die Entität übermitteln müssen.

Nach Ausführungsformen der Erfindung umfasst das Verwenden der TAN:
- Senden einer ersten Nutzerdatenanfrage beinhaltend die TAN an den Server durch das Entitätsgerät;
- Empfang einer ersten Nutzerdatenanfrage beinhaltend die TAN durch den Server von dem Entitätsgerät;
- In Antwort auf den Empfang der Nutzerdatenanfrage, senden des verschlüsselten Tracinggeheimnis-Transferobjekts, das mit der TAN verknüpft gespeichert ist, von dem Server an das Entitäts-Gerät;
- Entschlüsseln des Tracinggeheimnis-Transferobjekts mit dem privaten Entitätenschlüssel, der zu dem öffentlichen Schlüssel der Entität korrespondiert, um die User-ID und den zur Entschlüsselung der verschlüsselten Nutzerdaten des Nutzers erforderlichen U-ser-Data-Entschlüsselungsschlüssel zu erhalten, durch das Entitäten-Gerät;
- Verwenden der entschlüsselten User-ID, um eine zweite Nutzerdatenanfrage beinhaltend die User-ID zu erzeugen und vom Entitäten-Gerät an den Server zu senden;
- Empfang der verschlüsselten Nutzerdaten des Nutzers vom Server durch das Entitäten-Gerät in Antwort auf die zweite Anfrage; und
- Entschlüsselung der verschlüsselten Nutzerdaten mittels des in dem Tracinggeheimnis-Transferobjekt enthaltenen Nutzerdaten-Entschlüsselungsschlüssels durch das Entitäten-Gerät.

Nach Ausführungsformen der Erfindung enthält das Tracinggeheimnis-Transferobjekt zusätzlich einen im Zuge der Registrierung des gegenüber dem Server erzeugten und zugriffsgeschützt ausschließlich im Nutzergerät gespeicherten Zufallswert ("userTracingSecret"). Der Server ist dazu konfiguriert, die Kandidaten-Trace-IDs mittels dieses Zufallswerts auf Basis einer Vielzahl von Zeitpunkten innerhalb des zurückliegenden Zeitraums und der U-ser-ID zu berechnen.

Dies kann vorteilhaft sein, da das Amt vor Empfang des Tracinggeheimnis-Transferobjekts mit dem userTracingSecret die Kandidaten-Trace-IDs nicht berechnen und somit auch nicht als Suchwert in den Daten des Servers verwenden kann, um Check-in Datensätze zu identifizieren, die zu dem betreffenden Nutzer gehören. Somit kann das Amt ohne die willentliche Freigabe und Bereitstellung des Tracinggeheimnis-Transferobjekts nicht ermittelt, wann und bei welchen Venues der Nutzer einen Check-in durchgeführt hat.

Nach Ausführungsformen enthält das Tracinggeheimnis-Transferobjekt zusätzlich ein im Zuge der Registrierung des Nutzers des gegenüber dem Server erzeugten und zugriffsgeschützt ausschließlich im Nutzergerät gespeicherten Zufallswert, der im Folgenden als userVerificationSecret bezeichnet wird. Der Server ist dazu konfiguriert:
- die auf dem Server gespeicherten Trace-IDs, die identisch zu einer der Kandidaten-Trace-IDs sind, zu ermitteln;
- das im Tracinggeheimnis-Transferobjekt enthaltene userVerificationSecret zu verwenden, um mit diesem einen Verifikationsdatenwert für jede der ermittelten Trace-IDs als Funktion der Trace-ID und der mit diesen ermittelten Trace-IDs verknüpft gespeicherten verschlüsselten Ausweis-Daten (408) zu berechnen;
- den für eine jede der ermittelten Trace-IDs jeweils berechneten Verifikationsdatenwert mit einem Verifikationsdatenwert zu vergleichen, der im Zuge des Check-ins verknüpft mit der ermittelten Trace-ID gespeichert wurde; und
- ein Ergebnis dieses Vergleichs an das zumindest eine Entitäten-Gerät zu kommunizieren.

Das zumindest eine Entitäten-Gerät ist dazu konfiguriert, im Falle des Ergebnisses, dass der Vergleich eine fehlende Übereinstimmung der Verifikationsdatenwerte ergibt, die Zuweisung der Trace-ID an den Nutzer als manipuliert zu behandeln.

Die Erstellung des userVerificationSecret im Zuge des Registrationsprozesses des Nutzers, die Verwendung dieses userVerificationSecrets zur Berechnung eines Verifikationsdatenwertes, der als Bestandteil eines Check-in Nachricht bzw. QR-Codes direkt oder indirekt vom Nutzergerät an den Server übermittelt wird und die Bereitstellung des userVerificationSecret als Bestandteil des Tracinggeheimnis-Transferobjekts ans Amt kann den Vorteil haben, dass dem Amt die Prüfung ermöglicht wird, ob ein bestimmter Check-in Datensatz vom gleichen Nutzergerät (und damit dem gleichen Nutzer) erzeugt wurde, welches aktuell auch das Tracinggeheimnis-Transferobjekt übermittelt hat. Falls also eine Trace-ID von einem Angreifer bei der Übertragung abgefangen und auf einem Nutzergerät des Angreifers für einen Check-in unter falscher Identität verwendet werden sollte, würde dies auffallen, denn das Nutzergerät des Angreifers könnte zwar die gestohlene Trace-ID nochmals für den betrügerischen Check-in verwenden, aber es hätte ein anderes userVerificationSecret als das "berechtigte" Nutzergerät (oder auch gar kein userVerificationSecret). Falls es dem Angreifer also gelingen sollte, einen Check-in Vorgang mit der gestohlenen Trace-ID durchzuführen, wäre diesen falschen Check-in Daten auf dem Server ein falscher Verifikationsdatenwert zugeordnet (oder gar keiner) und ein Vergleich mit dem Verifikationsdatenwert aus dem Tracinggeheimnis-Transferobjekt würde fehlende Übereinstimmung ergeben.

Nach Ausführungsformen der Erfindung ist das elektronische System, und insbesondere der Server, so konfiguriert ist, dass diejenigen Nutzer ermittelt werden, die ebenfalls einen oder mehrere der ermittelten Venues während des vergangenen Zeitraums und während einer Aufenthaltsdauer des Nutzers besucht haben. Hierzu werden diejenigen der Check-in Datensätze ermittelt (sowie deren Trace-IDs), die eine Venue-ID und einen Check-in Zeitstempel enthalten aus welchen hervorgeht, dass sich die jeweiligen Check-in-Ereignisse an einem der ermittelten Venues während eines Zeitraums ergeben haben, an dem sich auch der zu tracende Nutzer in der Venue aufgehalten hat.

Durch Entschlüsselung der mit dem Entitätenschlüssel verschlüsselten Ausweisdaten dieser ermittelten Check-in Datensätze erlangt die Entität die Entschlüsselungsschlüssel für die Nutzerdaten der möglichen Kontaktpersonen, und somit an die Kontaktdaten der möglichen Kontaktpersonen. Optional haben die Venue-Geräte die Ausweisdaten in den von diesen erzeugten Check-in Datensätzen zusätzlich mit einem Schlüssel des Venue-Geräts verschlüsselt, sodass in diesem Fall der Server zunächst die ermittelten Check-in Datensätze an das durch die Venue-ID identifizierte Venue-Gerät senden muss um dieses die Ausweis-Daten entschlüsseln zu lassen und die entschlüsselten Ausweisdaten, die immer noch mit dem Entitätenschlüssel verschlüsselt sind, über den Server dem Entitäten-Gerät zur Verfügung zu stellen.

In einem weiteren Aspekt betrifft die Erfindung ein elektronisches System, das nur eine oder einige der Komponenten des oben beschriebenen Systems umfasst, z.B. nur den Server, nur das Nutzergerät, nur ein Entitätengerät, nur ein Venue-Gerät, oder z.B. eine Kombination aus dem Server und einem Nutzergerät, eine Kombination aus dem Server und dem Venue-Gerät, eine Kombination aus dem Server und dem Entitäten-Gerät, oder eine Kombination aus dem Venue-Gerät oder dem Venue-Objekt und dem Nutzergerät.

In einem weiteren Aspekt betrifft die Erfindung ein digitales Speichermedium mit von einem Prozessor eines Nutzergeräts ausführbaren Programminstruktionen zur Durchführung derjenigen Schritte zu deren Durchführung des elektronische System nach einer der hier beschriebenen Ausführungsformen der Erfindung konfiguriert ist, welche das Nutzergerät betreffen.

In einem weiteren Aspekt betrifft die Erfindung ein digitales Speichermedium mit von einem Prozessor ausführbaren Programminstruktionen zur Durchführung derjenigen Schritte zu deren Durchführung des elektronische System nach einer der hier beschriebenen Ausführungsformen der Erfindung konfiguriert ist, welche den Server betreffen.

In einem weiteren Aspekt betrifft die Erfindung ein digitales Speichermedium mit von einem Prozessor ausführbaren Programminstruktionen zur Durchführung derjenigen Schritte zu deren Durchführung des elektronische System nach einer der hier beschriebenen Ausführungsformen der Erfindung konfiguriert ist, welche das zumindest eine Venue-Gerät betreffen.

In einem weiteren Aspekt betrifft die Erfindung ein digitales Speichermedium mit von einem Prozessor ausführbaren Programminstruktionen zur Durchführung derjenigen Schritte zu deren Durchführung des elektronische System nach einer der hier beschriebenen Ausführungsformen der Erfindung konfiguriert ist, welche das zumindest eine Entitäten-Gerät betreffen.

In einem weiteren Aspekt betrifft die Erfindung ein Nutzergerät eines Nutzers, das dazu verwendet werden kann um den Nutzer, der sich temporär in verschiedenen räumlichen Bereichen (Venues) aufhalten kann, für eine Entität (246) zu tracen, wobei das Nutzergerät dazu ausgebildet ist:
- anlässlich einer Registrierung des Nutzers: Verschlüsselung von Nutzerdaten des Nutzers mit einem für den Nutzer individuellen User-Data-Key; und Speicherung der verschlüsselten Nutzerdaten verknüpft mit einer User-ID des Nutzers auf dem Server zu speichern; und Speicherung der User-ID und eines Nutzer-individuellen Entschlüsse-Iungsschlüssels, der zur Entschlüsselung der verschlüsselten Nutzerdaten erforderlich ist (kann im symmetrischen Fall der UserDataKey oder im asymmetrischen Fall ein zu diesem korrespondierender privater Schlüssel sein) in dem Nutzergerät, wobei vorzugsweise dieser Schlüssel geschützt gespeichert wird und dem Server nicht zugänglich ist;
- Im Zuge eines Check-ins des der Nutzer gegenüber einem der Venues, Erzeugung einer Trace-ID, wobei die Trace-ID repräsentativ ist für eine Kombination aus der User-ID des einen Nutzers und einer aktuellen Zeitinformation, und Übermittlung eines Check-in Datensatzes mit der Trace-ID an ein Venue-Gerät oder Übermittlung des Check-in Datensatzes beinhaltend eine Venue-ID des einen Venue auf den Server, um die Speicherung der Trace-ID mit den mit ihr verknüpften Daten (Check-in Daten) auf dem Server zu ermöglichen, wobei vorzugsweise die Trace-ID als Zugriffsschlüssel gespeichert wird,
- Optional eine Funktion zum Erfassen eines graphischen Codes auf einem Trägerobjekt mit einer Kamera des Nutzergeräts zur Anbahnung eines Check-ins, zur Extraktion einer Venue-ID aus dem Code und zur Verknüpfung der extrahierten Venue-ID mit der Trace-ID, wobei der Check-in Datensatz mit der extrahierten Venue-ID direkt an den Server gesendet wird;
- Optional eine Funktionalität zur Erstellung eines Tracinggeheimnis-Transferobjekts in Reaktion auf eine Nutzer-Interaktion mit dem Nutzergerät und zur Übermittlung des Tracinggeheimnis-Transferobjekts an den Server;
- Optional eine Schnittstelle zum Server zum Erhalt einer TAN, die mit dem Tracinggeheimnis-Transferobjekt verbunden ist, von dem Server, wobei das Nutzergerät zur Anzeige oder anderweitigen Ausgabe der empfangenen TAN an den Nutzer konfiguriert ist.

In einem weiteren Aspekt betrifft die Erfindung einen Server, das dazu verwendet werden kann um einen jeden von einer Vielzahl von Nutzern, die bei dem Server registriert sind, der sich temporär in verschiedenen räumlichen Bereichen (Venues) aufgehalten hat, für eine Entität zu tracen, wobei der Server ausgebildet ist zum:
- Speichern von verschlüsselten Nutzerdaten, die zumindest mit einem nutzerindividuellen Verschlüsselungsschlüssel und optional auch mit einem Entitäten-Schlüssel verschlüsselt sind, im Zuge der Registrierung jedes der Nutzer in einer Datenbank;
- Empfang eines Check-in Datensatzes im Zuge des Check-ins des einen Nutzers gegenüber einem Venue-Gerät von dem Nutzergerät des Nutzers oder von dem Venue-Gerät, wobei der Check-in Datensatz eine Trace-ID umfasst sowie eine Venue-ID des Venues sowie Ausweisdaten, wobei zumindest die Ausweisdaten mit einem Schlüssel der Entität verschlüsselt sind, wobei die Ausweisdaten zumindest die User-ID des Nutzers sowie einen nutzerspezifischen Schlüssel zur Entschlüsselung der Nutzerdaten dieses Nutzers beinhalten, wobei die Trace-ID repräsentativ ist für eine Kombination aus der User-ID des Nutzers und einer aktuellen Check-in Zeitinformation,
- Speichern des empfangenen Check-in Datensatzes in der Datenbank, wobei vorzugsweise die Trace-ID als Zugriffsschlüssel gespeichert wird.

Nach Ausführungsformen ist der Server ferner ausgebildet zum:
- Empfang einer Mitteilung beinhaltend die User-ID eines der Nutzer, bezüglich welchem das Tracing durchgeführt werden soll;
- Erzeugung von Kandidaten-Trace-IDs für den in der Mitteilung identifizierten Nutzer, wobei die Kandidaten-Trace-IDs mögliche zurückliegende Trace-IDs sind, die innerhalb eines vorgegebenen vergangenen Zeitraums liegen; vorzugsweise enthält die Mitteilung aller zur Berechnung der Kandidaten-Trace-IDs nötigen Daten, also insb. die UserID und das TracingSecret, und vorzugsweise auch eine Spezifikation des Zeitraums, für welchen die Kandidaten-Tracing-IDs berechnet werden sollen; Gemäß einer alternativen Ausführungsform empfängt der Server die Kandidaten-Trace-IDs stattdessen von einem Entitäten-Gerät der Entität;

- Verwendung der erzeugten oder empfangenen Kandidaten-Trace-IDs als Suchkriterium , um entsprechende auf dem Server für den vergangenen Zeitraum gespeicherte Trace-IDs dieses Nutzers aufzufinden, so dass die Venue-IDs derjenigen Venues gegenüber welchen der zu tracende Nutzer während des vergangenen Zeitraums einen Check-in durchgeführt hat, ermittelt werden, und
- Ausgabe der Venue-IDs der ermittelten Venues zur Identifizierung derjenigen Venues, die der zu tracende Nutzer während des vergangenen Zeitraums besucht hat, durch den Server.

In einem weiteren Aspekt betrifft die Erfindung ein Datenverarbeitungsgerät, das einer Entität, insbesondere einem Amt oder einer Behörde, zugewiesen ist, und als Entitäten-Gerät bezeichnet wird. Das Entitäten-Gerät kann dazu verwendet werden, einen jeden von einer Vielzahl von Nutzern, die bei einem Tracing-Server registriert sind, und die sich temporär in verschiedenen räumlichen Bereichen (Venues) aufgehalten haben, für die Entität zu tracen, wobei das Entitäten-Gerät ausgebildet ist zum:
- Empfang einer Mitteilung beinhaltend die User-ID eines der Nutzer, bezüglich welchem das Tracing durchgeführt werden soll;
- Erzeugung von Kandidaten-Trace-IDs für den in der Mitteilung identifizierten Nutzer, wobei die Kandidaten-Trace-IDs mögliche zurückliegende Trace-IDs sind, die innerhalb eines vorgegebenen vergangenen Zeitraums liegen; vorzugsweise enthält die Mitteilung aller zur Berechnung der Kandidaten-Trace-IDs nötigen Daten, also insb. die NutzerlD und das TracingSecret, und vorzugsweise auch eine Spezifikation des Zeitraums, für welchen die Kandidaten-Tracing-IDs berechnet werden sollen;
- Senden der erzeugten oder empfangenen Kandidaten-Trace-IDs als Bestanteil einer Suchanfrage an den Server, wobei die Anfrage die Kandidaten-Trace-IDs als Suchkriterium enthält, um entsprechende auf dem Server für den vergangenen Zeitraum gespeicherte Trace-IDs dieses Nutzers aufzufinden, so dass die Venue-IDs derjenigen Venues gegenüber welchen der zu tracende Nutzer während des vergangenen Zeitraums einen Check-in durchgeführt hat, ermittelt werden, und

- Empfang der Venue-IDs der ermittelten Venues zur Identifizierung derjenigen Venues, die der zu tracende Nutzer während des vergangenen Zeitraums besucht hat, von dem Server über ein Netzwerk.

In einem weiteren Aspekt betrifft die Erfindung ein Datenverarbeitungsgerät, das einem Venue, also einem räumlichen Bereich, in dem sich Nutzer aufhalten können, zugewiesen ist, und als Venue-Gerät bezeichnet wird. Das Venue-Gerät kann dazu verwendet werden, einem jeden von einer Vielzahl von Nutzern, die bei einem Tracing-Server registriert sind, und die sich temporär in verschiedenen räumlichen Bereichen (Venues) aufhalten können, einen automatischen oder semi-automatischen Check-in in den Venue zu ermöglichen, wobei der Besuch des Venues für die Entität tracebar ist, wobei das Venue-Gerät eine Schnittstelle zur lokalen Datenübertragung mit einem Nutzergerät eines Nutzers, der einen Check-in gegenüber dem Venue vornehmen will, beinhaltet und ausgebildet ist zum:
- Empfang eines Check-in Signals (z.B. QR Code oder Nahfeldsignal) von dem Nutzergerät des Nutzers, wobei das Check-in Signal einen Check-in Datensatz beinhaltet, wobei der Check-in Datensatz zumindest eine Trace-ID, eine Check-in Zeitinformation und verschlüsselte Ausweisdaten des Nutzers beinhaltet, wobei die Ausweisdaten mit einem Verschlüsselungsschlüssel der Entität verschlüsselt sind und zumindest die User-ID des Nutzers und einen für diesen Nutzer spezifischen Entschlüsselungsschlüssel für die Nutzerdaten dieses Nutzers beinhalten;
- Optional: Prüfung des Alters der Zeitinformation in dem Check-in Datensatz und Weiterverarbeitung der Check-in Datensatzes nur dann, wenn die Zeitinformation ein Maximalalter nicht überschreitet;
- Ergänzung der empfangenen Check-in Daten um die Venue-ID des Venue;
- Optional: Verschlüsseln zumindest eines Teils des empfangenen und ergänzten Check-in Datensatzes mit einem Venue-spezifischen Verschlüsselungsschlüssel, wobei die TracelD unverschlüsselt bleibt;
- Übermittlung der ergänzten und optional mit dem Venue-Schlüssel verschlüsselten Check-in Daten über das Netzwerk an den Server.

In einem weiteren Aspekt betrifft die Erfindung ein computerimplementiertes Verfahren zum Tracing von Nutzern, die sich temporär in verschiedenen räumlichen Bereichen (Venues) aufhalten, für eine Entität, insbesondere für Notfallszenarien wie in einer Pandemie. Das Verfahren umfasst:
- anlässlich einer Registrierung eines jeden der Nutzer: Speicherung von Nutzerdaten des Nutzers auf einem Server, wobei die Nutzerdaten von einem dem Nutzer zugeordneten Nutzergerät mit einem für den Nutzer individuellen User-Data-Key verschlüsselt werden, wobei insbesondere eine User-ID des Nutzers verknüpft mit den verschlüsselten Nutzerdaten auf dem Server gespeichert werden; und Speicherung der User-ID und eines Nutzer-individuellen zur Entschlüsselung der verschlüsselten Nutzerdaten erforderlichen User-Data-Entschlüsselungsschlüssels in dem Nutzergerät, wobei vorzugsweise der User-Data-Key und/oder der User-Data-Entschlüsselungsschlüssel geschützt gespeichert werden und dem Server nicht zugänglich sind;
- Im Zuge eines Check-ins eines der Nutzer gegenüber einem der Venues, Erzeugung einer Trace-ID durch das Nutzergerät des einen Nutzers, wobei die Trace-ID repräsentativ ist für eine Kombination aus der User-ID des einen Nutzers und einer aktuellen Zeitinformation, und Speicherung der Trace-ID verknüpft mit der Venue-ID des einen Venue auf dem Server, wobei die Trace-ID als Zugriffsschlüssel gespeichert wird,
- Empfang einer Mitteilung beinhaltend die User-ID eines der Nutzer, bezüglich welchem das Tracing durchgeführt werden soll;
- Erzeugung von Kandidaten-Trace-IDs für den in der Mitteilung identifizierten Nutzer, wobei die Kandidaten-Trace-IDs mögliche zurückliegende Trace-IDs sind, die innerhalb eines vorgegebenen vergangenen Zeitraums liegen,
- Verwendung der erzeugten Kandidaten-Trace-IDs als Suchkriterium durch den Server, um entsprechende auf dem Server für den vergangenen Zeitraum gespeicherte Trace-IDs aufzufinden, so dass die Venue-IDs derjenigen Venues gegenüber welchen der zu tracende Nutzer während des vergangenen Zeitraums einen Check-in durchgeführt hat, ermittelt werden, und
- Ausgabe der Venue-IDs der ermittelten Venues zur Identifizierung derjenigen Venues, die der zu tracende Nutzer während des vergangenen Zeitraums besucht hat, durch den Server.

Nach Ausführungsformen umfasst das Verfahren weitere Schritte, die von ein oder mehreren Komponenten eines elektronischen Systems zum Tracing von Nutzern ausgeführt werden, und die bereits im Hinblick auf diese Ausführungsvarianten des elektronischen Systems beschrieben wurden.

In einem weiteren Aspekt betrifft die Erfindung ein elektronisches System mit Nutzergeräten, die jeweils einem Nutzer zu-geordnet sind, Venue-Geräten und zumindest einem Server zum Tracing von Nutzern für eine Entität, der ein asymmetrisches kryptographisches Schlüsselpaar mit einem öffentlichen und einem privaten Entitätenschlüssel zugeordnet ist, wobei das elektronische System zur Durchführung der folgenden Schritte konfiguriert ist:
- Speicherung von Nutzerdaten der Nutzer anlässlich einer Registrierung auf einem Server, wobei die Nutzerdaten durch den öffentlichen Entitätenschlüssel verschlüsselt sind und das Resultat dieser Ver-schlüsselung mit einem jeweiligen User-Data-Key des betreffenden Nutzers verschlüsselt ist,
- Speicherung in jedem der Nutzergeräte eines User-Data-Key, eines User-Secret und einer User-ID, wobei das Nutzergerät über eine Netzwerkschnittstelle Zugriff auf eine aktuelle Zeitinformation hat und das Nutzergerät konfiguriert ist zur Erzeugung einer Trace-ID, die repräsentativ ist für eine Kombination aus User-ID, User-Secret und der aktuellen Zeitinformation sowie zur Erzeugung eines Chiffrats aus dem User-Data-Key und der User-ID, wobei der öffentliche Entitäten-schlüssel zur Erzeugung des Chiffrats verwendet wird, und wobei das Nutzergerät eine Kommunikationsschnittstelle zur direkten Kommunikation mit einem Venue-Gerät aufweist, über welche die Trace-ID und das Chiffrat an das Venue-Gerät übertragen werden,

- Erfassung eines der Nutzer durch ein Venue-Gerät mit Hilfe des Nutzergeräts des Nutzers, wobei das Venue-Gerät eine Schnittstelle für die direkte Kommunikation mit der entsprechenden Schnittstelle des Nutzergeräts aufweist, und mit einer Netzwerkschnittstelle zur Kommunikation mit dem Server, wobei das Venue-Gerät dazu konfiguriert ist, zur Erfassung des Nutzers die Trace-ID und das Chiffrat von dem Nutzergerät zu empfangen und zu prüfen, ob die Zeitinformation einer empfangenen Trace-ID hinreichend aktuell ist, und wenn dies der Fall ist, die Trace-ID mit einer dem Venue-Gerät zugeordneten Venue-ID und dem Chiffrat an den Server zu übertragen,
- Speicherung einer empfangenen Trace-ID und dem Chiffrat mit der Zeitinformation als Zugriffsschlüssel durch den Server,
- Empfang einer Mitteilung der User-ID und des User-Secrets von einem der Nutzer an die Entität,
- Erzeugung von möglichen zurückliegenden Trace-IDs für den betreffenden Nutzer, die innerhalb eines vorgegebenen vergangenen Zeitraums liegen und Verwendung der erzeugten Trace-IDs als Suchkriterium, um entsprechende auf dem Server für den vergangenen Zeitraum gespeicherte Trace-IDs aufzufinden, so dass die Venue-IDs derjenigen Venues von denen der Nutzer während des vergangenen Zeitraums erfasst wurde, ermittelt werden,
- Ausgabe der Venue-IDs der ermittelten Venues zur Identifizierung derjenigen Venues, die der Nutzer während des vergangenen Zeitraums besucht hat.

Nach Ausführungsformen ist das elektronische System so konfiguriert, dass von dem Nutzergerät des einen der Nutzer eine Check-out Zeit an den Server übertragen wird, wenn der Nutzer den Venue verlässt, wobei die Check-out Zeit der zuvor für diesen Nutzer empfangenen Trace-ID zugeordnet wird, so dass für den Nutzer der Aufenthaltszeitraum an dem Venue gespeichert wird.

Nach Ausführungsformen ist das elektronische System so konfiguriert, dass diejenigen Nutzer ermittelt werden, die ebenfalls einen oder mehrere der ermittelten Venues während des vergangenen Zeitraums und während einer Aufenthaltsdauer des Nutzers besucht haben, wozu diejenigen der Trace-IDs, die von den ermittelten Venues während des vergangenen Zeitraums zu dem Server übertragen wurden, ermittelt werden.

In einem weiteren Aspekt betrifft die Erfindung ein digitales Speichermedium mit von einem Prozessor eines Datenverarbeitungsgeräts ausführbaren Programminstruktionen zur Durchführung derjenigen Schritte zu deren Durchführung des elektronische System nach einer der hier beschriebenen Ausführungsformen, welche das Datenverarbeitungsgerät betreffen, wobei es sich bei dem Datenverarbeitungsgerät um das Nutzergerät, ein Entitäten-Gerät, ein Venue-Gerät oder um den Server handelt.

In einem weiteren Aspekt betrifft die Erfindung ein elektronisches System mit Nutzergeräten, die jeweils einem Nutzer zugeordnet sind, Venue-Geräten und zumindest einem Server zum Tracing von Nutzern für eine Entität, der ein asymmetrisches kryptographisches Schlüsselpaar mit einem öffentlichen und einem privaten Entitätenschlüssel zugeordnet ist, wobei das elektronische System zur Durchführung der folgenden Schritte konfiguriert ist:
- Speicherung von Nutzerdaten der Nutzer anlässlich einer Registrierung auf einem Server, wobei die Nutzerdaten durch den öffentlichen Entitätenschlüssel verschlüsselt sind,
- Speicherung einer User-ID in jedem der Nutzergeräte, wobei das Nutzergerät über eine Netzwerkschnittstelle Zugriff auf eine aktuelle Zeitinformation hat und das Nutzergerät konfiguriert ist zur Erzeugung einer Trace-ID, die repräsentativ ist für eine Kombination aus User-ID und der aktuellen Zeitinformation und wobei das Nutzergerät eine Kommunikationsschnittstelle zur direkten Kommunikation mit einem Venue-Gerät aufweist, über welche die Trace-ID an das Venue-Gerät übertragen werden,

- Erfassung eines der Nutzer durch ein Venue-Gerät mit Hilfe des Nutzergeräts des Nutzers, wobei das Venue-Gerät eine Schnittstelle für die direkte Kommunikation mit der entsprechenden Schnittstelle des Nutzergeräts aufweist, und mit einer Netzwerkschnittstelle zur Kommunikation mit dem Server, wobei das Venue-Gerät dazu konfiguriert ist, zur Erfassung des Nutzers die Trace-ID von dem Nutzergerät zu empfangen und zu prüfen, ob die Zeitinformation einer empfangenen Trace-ID hinreichend aktuell ist, und wenn dies der Fall ist, die Trace-ID mit einer dem Venue-Gerät zugeordneten Venue-ID an den Server zu übertragen,
- Speicherung einer empfangenen Trace-ID mit der Zeitinformation als Zugriffsschlüssel durch den Server,
- Empfang einer Mitteilung der User-ID von einem der Nutzer an die Entität,
- Erzeugung von möglichen zurückliegenden Trace-IDs für den betreffenden Nutzer, die innerhalb eines vorgegebenen vergangenen Zeitraums liegen und Verwendung der erzeugten Trace-IDs als Suchkriterium, um entsprechende auf dem Server für den vergangenen Zeitraum gespeicherte Trace-IDs aufzufinden, so dass die Venue-IDs derjenigen Venues von denen der Nutzer während des vergangenen Zeitraums erfasst wurde, ermittelt werden,
- Ausgabe der Venue-IDs der ermittelten Venues zur Identifizierung derjenigen Venues, die der Nutzer während des vergangenen Zeitraums besucht hat.

Nach Ausführungsformen ist das elektronische System so konfiguriert, dass von dem Nutzergerät des einen der Nutzer oder von dem Venue-Gerät eine Check-out Zeit an den Server übertragen wird, wenn der Nutzer den Venue verlässt, wobei die Check-out Zeit der zuvor für diesen Nutzer empfangenen Trace-ID zugeordnet wird, so dass für den Nutzer der Aufenthaltszeitraum an dem Venue gespeichert wird.

Nach Ausführungsformen ist das elektronische System so konfiguriert, dass diejenigen Nutzer ermittelt werden, die ebenfalls einen oder mehrere der ermittelten Venues während des vergangenen Zeitraums und während einer Aufenthaltsdauer des Nutzers besucht haben, wozu diejenigen der Trace-IDs, die von den ermittelten Venues während des vergangenen Zeitraums zu dem Server übertragen wurden, ermittelt werden.

In einem weiteren Aspekt betrifft die Erfindung ein digitales Speichermedium mit von einem Prozessor eines Datenverarbeitungsgeräts ausführbaren Programminstruktionen zur Durchführung derjenigen Schritte zu deren Durchführung des elektronische System nach einer der hier beschriebenen Ausführungsformen, welche das Datenverarbeitungsgerät betreffen, wobei es sich bei dem Datenverarbeitungsgerät um das Nutzergerät, ein Entitäten-Gerät, ein Venue-Gerät oder um den Server handelt.

Unter einem "Nutzergerät" wird hier ein portables Datenverarbeitungssystem verstanden, insbesondere ein batteriegetriebenes Datenverarbeitungssystem wie z.B. ein Nutzergerät, ein Notebook, ein Tablet-Computer oder ein integriertes Gerät, das spezifisch für das automatische Check-in und Tracing von Besuchern an einem Venue konzipiert wurde und/oder genutzt wird.

Unter einem "Mobilfunkgerät" wird hier ein portables elektronisches System, insbesondere ein batteriegetriebenes Telekommunikationsgerät verstanden, welches über zumindest einen Prozessor zur Ausführung von Programminstruktionen verfügt, insbesondere zur Ausführung von sogenannten Apps. Ein Mobilfunkgerät hat eine Netzwerkschnittstelle zum Aufbau von Kommunikationsverbindungen über ein Telekommunikationsnetzwerk, welches nach einem Mobilfunkstandard ausgebildet sein kann.

Das Nutzergerät kann optional über weitere Kommunikationsschnittstellen verfügen, wie zum Beispiel eine Anzeigevorrichtung, das heißt ein sogenanntes Display, für eine optische Kommunikation mit dem Nutzer oder auch für eine maschinelle Kommunikation beispielsweise durch Anzeigen optischer Muster, wie zum Beispiel eines QR-Codes. Über diese weitere Kommunikationsschnittstelle erfolgt die Kommunikation direkt, das heißt ohne Zwischenschaltung eines Netzwerks. Beispielsweise weist das Nutzergerät hierzu auch ein oder mehrere Nahfeldkommunikationsschnittstellen auf, beispielsweise nach dem Bluetooth- und/oder NFC-Standard. Das Nutzergerät verfügt ferner über zumindest einen Speicher, in dem Daten gespeichert werden können.

Ein Nutzergerät ist einem Nutzer eindeutig zugeordnet. Dies erfolgt oftmals, insbesondere bei Mobilfunkgeräten, über ein SIM oder eSIM.

Unter einem "Venue"-Gerät wird hier insbesondere ein elektronisches Gerät verstanden, welches der Betreiber eines Venues, das heißt eines Veranstaltungsorts für eine öffentliche oder private Veranstaltung oder einer Gaststätte, wie zum Beispiel Restaurant, Club oder dergleichen, verwendet, um die Nutzer, welche den Venue besuchen, zu erfassen. Ein Venue kann auch ein Fortbewegungsmittel oder ein Bereich in diesem sein, z.B. ein Flugzeug, ein Zug oder Zugabteil. Ein Venue-Gerät kann als monolithisches oder verteiltes Datenverarbeitungssystem ausgebildet sein, z.B. als Mobilfunkgerät oder als stationärer Terminal, wie zum Beispiel als PC. Bei einem Venue-Gerät kann es sich auch um eine automatisierte Zugangskontrollvorrichtung, beispielsweise mit einem Drehkreuz oder einer Zugangsschleuse, handeln. Das Venue-Gerät kann auch ein Datenverarbeitungssystem eines Drittanbieters sein, z.B. für Zwecke des Ticketings (automatisches Bezahlen, Besucherverwaltung, etc.) Das Venue-Gerät verfügt über eine Schnittstelle für die direkte Kommunikation mit der entsprechenden Schnittstelle des Nutzergeräts sowie über eine Netzwerkschnittstelle zur Kommunikation mit einem Server über ein Telekommunikationsnetzwerk, welches drahtgebunden oder drahtlos ausgebildet sein kann.

Unter einem "Server" wird hier ein Datenverarbeitungssystem verstanden, insbesondere ein Servercomputer, ein virtueller Server oder ein verteilter Server, der beispielsweise über Cloudcomputing realisiert ist. Der Server dient zum Tracing der Nutzer, wobei hier unter "Tracing" die Möglichkeit der Rückverfolgung von in der Vergangenheit liegenden Besuchen eines Nutzers an Venues verstanden wird sowie optional auch die Möglichkeit, diejenigen Nutzer zu ermitteln, welche sich gleichzeitig mit einem bestimmten Nutzer, der beispielsweise mit Covid-19 infiziert ist, an einem der Venues aufgehalten haben. Gemäß Ausführungsformen kann der Server auch Computer und/oder Funktionen von Drittanbietern integrieren und diese nutzen.

Unter einem "Nutzer" wird hier eine natürliche Person verstanden, die als Besucher einer oder mehrerer räumlicher Bereiche (Venues) agieren kann.

Unter dem Begriff "Tracing" wird hier die Nachverfolgung von Kontakten eines bestimmten Nutzers über einen Zeitraum verstanden, wobei vorzugsweise keine Bewegungsprofile erstellt werden oder werden müssen.

Dieses Tracing soll für eine "Entität" stattfinden. Unter einer "Entität" wird hier eine vertrauenswürdige natürliche oder juristische Person verstanden, insbesondere eine Institution, die z.B. hoheitlichen Rechte für die Ermittlung der Aufenthaltsorte der Nutzer hat, wie zum Beispiel das Gesundheitsamt im Falle einer Pandemie.

Erfindungsgemäß wird gemäß Ausführungsformen so vorgegangen, dass sich die Nutzer zunächst mithilfe derjeweiligen Nutzergeräte registrieren. Hierzu werden zunächst Nutzerdaten erfasst, also beispielsweise durch Eingabe der Nutzerdaten in das betreffende Nutzergerät oder durch Einlesen der Nutzerdaten, beispielsweise aus einem elektronischen Personalausweis des Nutzers in das Nutzergerät. Die Nutzerdaten werden dann mithilfe des öffentlichen Entitäten-schlüssels, also beispielsweise mithilfe eines öffentlichen Schlüssels des Gesundheitsamts, von dem Nutzergerät verschlüsselt und das Resultat dieser Verschlüsselung wird mit einem User-Data-Key des betreffenden Nutzers verschlüsselt, wobei es sich bei dem User-Data-Key hier beispielsweise um einen Zufallswert hoher Entropie handelt, wie zum Beispiel einer Länge von 16 Bytes.

Die Verschlüsselung der Nutzerdaten kann gemäß einer bevorzugten Ausführungsform unmittelbar durch den für den Nutzer im Zuge der Registrierung erzeugten User-Data-Key (nutzerspezifischer Verschlüsselungsschlüssel für die Nutzerdaten des Nutzers) erfolgen. Gemäß einer anderen Ausführungsform erfolgt die Verschlüsselung unmittelbar durch den öffentlichen Entitätenschlüssel. Gemäß einer weiteren alternativen Ausführungsform erfolgt die Verschlüsselung der Nutzerdaten durch eine zweifache sequenzielle Verschlüsselung zunächst mit dem User-Data-Key und dann mit dem öffentlichen Entitätenschlüssel oder umgekehrt. Gemäß einer weiteren Ausführungsform leitet das Nutzergerät aus dem öffentlichen Entitätenschlüssel ein weiterer Schlüssel nach einem vordefinierten Verfahren ab, welcher dann zur Verschlüsselung der Nutzerdaten dient. Beispielsweise kann der weitere Schlüssel aus dem öffentlichen Entitätenschlüssel und dem User-Data-Key abgeleitet werden.

Bei dem Entitätenschlüssel kann es sich um einen Master Key handeln oder den Schlüssel einer der Entität zugeordneten abhängigen Entität. Beispielsweise kann es sich im Fall des Gesundheitsamts um den Masterschlüssel des Gesundheitsamts oder um einen Schlüssel des jeweils vor Ort verantwortlichen Gesundheitsamts handeln.

Der User-Data-Key kann beispielsweise für die Zwecke der Registrierung durch das Betriebssystem des Nutzergeräts zur Verfügung gestellt werden. Beispielsweise verfügt das Nutzergerät über eine Binary Symmetric Source zur Erzeugung von Zufallswerten hoher Entropie.

Die Hinterlegung der Nutzerdaten auf dem Server erfolgt also in einer Form, die es dem Server zunächst nicht ermöglicht, diese Nutzerdaten zu entschlüsseln, da dies ohne den privaten Entitätenschlüssel und/oder den zu dem User-Data-Key korrespondierenden User-Data-Entschlüsselungsschlüssel nicht möglich ist.

Gemäß Ausführungsformen handelt es sich bei dem User-Data-Key um einen symmetrischen Schlüssel, d.h., der zur Verschlüsselung der Nutzerdaten verwendete User-Data Key und der zur Entschlüsselung verwendete User-Data-Entschlüsselungsschlüssel sind identisch. Gemäß einer alternativen Ausführungsform handelt es sich bei dem User-Data-Key und dem User-Data-Entschlüsselungsschlüssel um ein asymmetrisches kryptographisches Schlüsselpaar, wobei der User-Data-Key zur Verschlüsselung und der User-Data Entschlüsselungsschlüssel zur Entschlüsselung verwendetet wird. In dem Nutzergerät wird dieser User-Data-Key in einem nichtflüchtigen Speicher persistent gespeichert. Aufgrund der Registrierung auf dem Server erzeugt der Server eine User-ID, das heißt einen eindeutigen Identifikator für den betreffenden User, die der Server aufgrund der Registrierung an das Nutzergerät zurückgibt. Die User-ID wird in dem Nutzergerät in einem nichtflüchtigen Speicher persistent gespeichert. Um einen Nutzer beim Besuch einer Venue zu erfassen, wird z.B. wie folgt vorgegangen:
Das Nutzergerät erzeugt eine Trace-ID, die repräsentativ ist für eine Kombination aus der User-ID und einer aktuellen Zeitinformation. Bei der aktuellen Zeitinformation handelt es sich um die aktuelle Uhrzeit beim Check-in des Nutzers an dem Venue, wobei diese aktuelle Uhrzeit von dem Nutzergerät über das Netzwerk ermittelt werden. Vorzugsweise wird diese aktuelle Uhrzeit nach einem vordefinierten Rundungsverfahren gerundet, beispielsweise auf die volle Minute auf- oder abgerundet.

Außerdem erzeugt das Nutzergerät verschlüsselte Ausweis-Daten dadurch, dass z.B. die Nutzer-ID und der zur Entschlüsselung der Nutzerdaten erforderliche User-Data-Entschlüsselungsschlüssel mit dem öffentlichen Entitätenschlüssel verschlüsselt werden. Die verschlüsselten Ausweisdaten enthalten also nur eine Nutzer-ID anstelle der Nutzerdaten, sind also so klein, dass die verschlüsselten Ausweisdaten zusammen mit der Trace-ID und optionalen weiteren Daten schnell und effizient an das Venue-Gerät übermittelt werden können, z.B. dadurch, dass diese Daten in einem QR-Code kodiert und auf einem Display des Nutzergeräts angezeigt und von einer Kamera des Venue-Geräts erfasst werden. Die optionalen weiteren Daten umfassen z.B. dem Zeitstempel, der zur Ableitung der Trace-ID verwendet wurde, sowie ggf. einem Verifikationsdatenwert und/oder einer QR-Code Version.

Die besagten Informationen können auch bei einem automatischen Check-Out übermittelt werden, wobei hierbei vorzugsweise die gleiche Trace-ID wie beim Check-in verwendet wird, und die aktuell erfasste und an das Venue-Gerät übermittelte Zeit die Check-out Zeit repräsentiert. Das Venue-Gerät prüft gemäß Ausführungsformen der Erfindung zur Erfassung eines Nutzers beim Check-in, ob die Zeitinformation, auf der die Trace-ID beruht, hinreichend aktuell ist, und verwendet hierzu beispielsweise ebenfalls eine über ein Netzwerk bezogene Zeitinformation zur Synchronisierung mit der von dem Nutzergerät verwendeten Zeitbasis und/oder als Referenzwert zum Vergleich mit der vom Nutzergerät erhaltenen Zeitinformation. Die Zeitinformation gilt als hinreichend aktuell, wenn sie beispielsweise innerhalb eines vorgegebenen Toleranzbereichs liegt, also beispielsweise nicht älter ist als einige Sekunden oder Minuten. Gemäß einer Ausführungsform ist die Zeitinformation in der Trace-ID so kodiert, dass diese vom Venue-Gerät nicht aus der Trace-ID extrahiert werden kann. In diesem Fall kann das Nutzergerät zusätzlich zur der Trace-ID die Check-in Zeit, die als Basis für die Ableitung der Trace-ID verwendet wurde, an das Venue-Gerät übermitteln, sodass das Venue-Gerät diese zusätzliche Zeit mit einer Referenzzeit (z.B. der Systemzeit des Venue-Geräts oder einer von einem Zeit-Server über das Internet) verglichen kann. Wenn die Zeitinformation auf der die Trace-ID beruht hinreichend aktuell ist, so überträgt das Venue-Gerät gemäß Ausführungsformen die Trace-ID mit einer dem Venue-Gerät zugeordneten Venue-ID und vorzugsweise auch weiteren Daten wie insb. einem Chiffrat (also den mit dem öffentlichen Entitätenschlüssel verschlüsselten Ausweisdaten) und ggf. einem oder mehreren der weiteren Daten an den Server. Diese Informationen werden auf dem Server gespeichert, wobei vorzugsweise die Speicherung so erfolgt, dass die Trace-ID als Suchschlüssel dient. Beispielsweise kann ein durchsuchbarer Index in einer Datenbank über der Spalte "Trace-ID" erstellt werden, um ein effizientes Suchen in der Gesamtheit aller Check-in Datensätze der beim Server registrierten Nutzer gegenüber den beim Server registrierten Venues zu ermöglichen.

Vorzugsweise wird dies für eine möglichst große Anzahl von Nutzern, vorzugsweise für sämtliche Nutzer, durchgeführt, die eine Venue besuchen, um so möglichst lückenlos sämtliche Nutzer zu erfassen.

Nachträglich kann das Erfordernis bestehen, den oder die Nutzer, welche den Venue besucht haben, gegenüber der Entität offenzulegen. Dies ist beispielsweise dann der Fall, wenn einer der Nutzer positiv auf Covid-19 getestet wird. Dieser mit Covid-19 infizierte Nutzer meldet dies der Entität, hier dem Gesundheitsamt, und teilt der Entität seine User-ID und sein User-Secret mit. Anhand dieser Informationen werden dann sämtliche mögliche zurückliegende Trace-IDs, die zur Erfassung eines Nutzers durch ein Venue-Gerät für einen vorgegebenen vergangenen Zeitraum benutzt sein könnten, erzeugt. Die Erzeugung kann z.B. auf dem Server oder einem Entitäten-Gerät erfolgen, sofern die hierfür erforderlichen Daten vorhanden sind. Die Menge dieser möglichen Trace-IDs, auch als "Kandidaten-Trace-IDs" bezeichnet, ist typischerweise relativ begrenzt, da nicht sämtliche mögliche Zeiten innerhalb des relevanten Zeitraums in Frage kommen, sondern nur diejenigen, die aufgrund des vorgegebenen Rundungsverfahrens tatsächlich vorkommen können. Beispielsweise wird zum Zwecke eines Tracings eines Nutzers über einen Zeitraum von 2 Wochen bei Verwendung einer Ableit-Funktion, die auf Zeitwerten beruhen, die auf die Minute genau erfasst und abgerundet werden, 14 Tage x 24 Stunden x 60 Minuten = ca. 20.000 Kandidaten-Trace-IDs für eine bestimmte User-ID berechnet.

Diese Kandidaten-Trace-IDs werden dann als Suchkriterium verwendet, um entsprechend auf dem Server für den vergangenen Zeitraum gespeicherte Trace-IDs aufzufinden, sodass die Venue-IDs derjenigen Venues, von denen der Nutzer während des vergangenen Zeitraums erfasst wurde, ermittelt werden. Hierdurch erhält also das Gesundheitsamt die Venue-IDs der ermittelten Venus, die der Nutzer in dem vergangenen Zeitraum, beispielsweise zwei Wochen, in denen er möglicherweise bereits infektiös war, besucht hatte. Ein Abgleich von 20.000 Datenwerten über einen durchsuchbaren Datenbankindex kann auf Basis heutiger Datenbanktechnologie sehr rasch und in der Regel in Echtzeit durchgeführt werden.

Nach einer Ausführungsform der Erfindung ist das elektronische System so konfiguriert, dass von dem Nutzergerät eines durch ein Venue-Gerät erfassten Nutzers, also nach einem Check-in, eine Check-out Zeit an den Server übertragen wird, wenn der Nutzer den Venue verlässt. Diese Check-out-Zeit ist der zuvor für diesen Nutzer empfangenen Trace-ID zugeordnet, sodass serverseitig die Information vorliegt, in welchem Zeitraum sich der Nutzer an dem Venue aufgehalten hat. Alternativ erfolgt dies durch das Venue-Gerät.

Nach einer Ausführungsform der Erfindung erfolgt dieser Check-out durch manuelle Eingabe in das Nutzergerät oder automatisch durch sogenanntes Geofencing. In letzterem Fall wird die Check-out-Zeit mit der zugeordneten Trace-ID automatisch von dem Nutzergerät an den Server gesendet, sobald der Nutzer die Venue verlässt. Alternativ erfolgt dies durch das Venue-Gerät.

Vorzugsweise geht diese Information in die Ermittlung derjenigen Nutzer ein, die sich ebenfalls an dem Venue aufgehalten hatten, indem die Suche auf solche Nutzer beschränkt wird, die nicht nur den gleichen Venue besucht hatten, sondern dort auch in einem zumindest mit dem Aufenthaltszeitraum des erkannten Nutzers überlappenden Aufenthaltszeitraums gewesen sind. Beispielsweise können in den Check-in Datensätzen des Servers, die die Trace-IDs der Nutzer beinhalten, auch die Venue-IDs und Check-in und optional auch Check-Out Zeiten im Klartext angegeben werden, sodass die Check-in Datensätzen von Nutzern, die zur gleichen Zeit wie der Nutzer, auf den sich der Notfall bezieht, in einem bestimmten Venue waren, ermittelt werden können. Als Venue-ID kann beispielsweise der Name und/oder Adresse, ein Identifikator oder auch die geografischen Koordinaten des Venues dienen. Im Weiteren werden Ausführungsformen der Erfindung mit Bezugnahme auf die Zeichnungen näher erläutert.

Es zeigen:
- Figur 1: ein Flussdiagramm eines Verfahrens zum Tracing von Nutzern,
- Figur 2: ein Blockdiagramm eines verteilten elektronischen Systems zum Tracing von Nutzern,
- Figuren 3A-3C: verschiedene Beispiele für die Erzeugung und Speicherung von Nutzerdaten ("User-Data-Package"),
- Figuren 4A-3C: verschiedene Beispiele für den Inhalt eines Check-in QR-Codes,
- Figuren 5A-5C: verschiedene Beispiele für die Erzeugung und Speicherung von Check-in Daten, wie sie auf dem Server gespeichert sind,
- Figuren 6A, 6B: Beispiele für Venue-relevante Zusatzdaten,
- Figur 7: ein UML-Diagramm eines Beispiels für das Tracing-Verfahren.

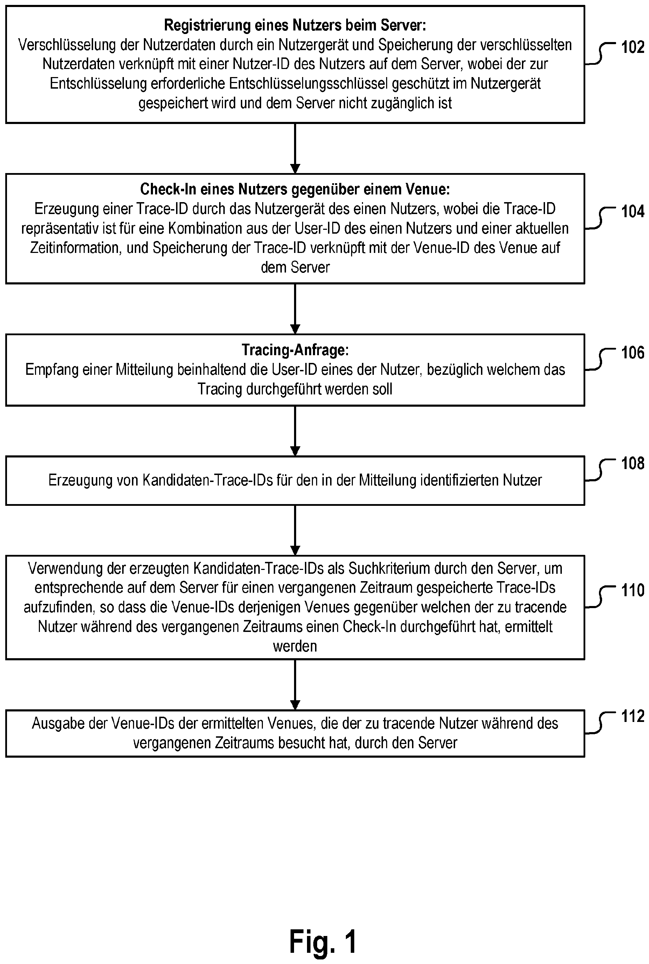

**Figur 1** zeigt ein Flussdiagramm eines Verfahrens zum Tracing von Nutzern. Nach Ausführungsform der Erfindung wird wie folgt vorgegangen:
a) **Registrierungsschritt** 102: Für den Nutzer werden eine Reihe von nutzer-individuellen Daten erzeugt, die teilweise beim Server, gegenüber welchem die Registrierung erfolgt, hinterlegt werden. Insbesondere wird für den Nutzer eine User-ID und ein nutzerspezifischer Verschlüsselungsschlüssel (User-Data-Key) sowie optional weitere Datenwerte erzeugt. Der User-Data-Key kann z.B. ein symmetrischer Schlüssel sein. Der Nutzer spezifiziert seine beim Server verschlüsselt zu hinterlegenden persönlichen Daten (Nutzerdaten) wie z.B. Name und Anschrift, Kontaktadresse, verschlüsselt die Nutzerdaten mithilfe seines Nutzergeräts, d. h. einer darauf ausgeführten App, seine schutzbedürftigen Daten mit seinem User-Data-Key verschlüsselt. Optional können die Nutzerdaten zusätzlich auch durch einen (vorzugsweise täglich neu erstellten) öffentlichen Entitätenschlüssel (zum Beispiel einem Gesundheitsamtsschlüssel oder je nach Ausführungsform einem daraus abgeleiteten Schlüssel) verschlüsselt sein. Gemäß einer weiteren Ausführungsvariante sind die Nutzerdaten nur mit dem öffentlichen Entitätenschlüssel verschlüsselt. Die Ausführungsvariante mit der User-Data-Key basierten Verschlüsselung ist jedoch sicherer und bietet einen besseren Datenschutz. Die App auf dem Nutzergerät lädt diese verschlüsselten schutzbedürftigen Daten auf den Server. Der Server ordnet den von dem Nutzergerät des Nutzers empfangenen verschlüsselten schutzbedürftigen Daten eine User-ID zu und gibt diese User-ID an die App des Nutzers zurück. Diese User ID wird von der App in einem Speicher des Nutzergeräts gespeichert.
b) **Check-in Schritt 104:** das Nutzergerät berechnet eine aktuelle Tracing-ID und übermittelt diese an ein Venue-Gerät zusammen mit anderen Check-in Daten, z.B. indem diese Daten in einem QR-Code kodiert und vom Venue-Gerät gescannt werden. Somit erhält das Venue-Gerät die Trace-ID von dem Nutzergerät des Nutzers und ggf. weitere Check-in Daten. Je nach Ausführungsform kann dies so erfolgen, dass die Trace-ID von dem Nutzergerät die User-ID im Klartext enthält wird oder in einer verschlüsselten bzw. verborgenen Form. In letzterem Fall kann der Server auf Basis der Trace-ID noch nicht einmal anonym einen Nutzer tracen; auch andere Erzeugungsverfahren für die Trace ID sind möglich, wenn man ein Tracing anhand der User-ID verhindern möchte. Das Venue-Gerät ergänzt zu der Trace-ID einen Zeitstempel, der die Check-in Zeit repräsentiert, und die Venue ID und schickt das (ggf. verschlüsselt) auf den Server. In anderen Ausführungsformen muss am Venue kein Venue-Gerät vorhanden sein. Das Nutzergerät liest die Venue-ID und ggf. weitere Venue-bezogene Daten ein (z.B. mittels eines QR-Codes auf einem Trägerobjekt oder über ein Nahfeldsignal), führt den Check-in mit einem den Venue repräsentierenden Modul der App durch und sendet die so erzeugten Check-in Daten mit der Venue-ID direkt an den Server.
c) Check-out-Schritt: von Venue oder dem Nutzergerät wird ein Check-out timestamp an den Server geschickt und der User-ID (oder einem Chiffrat davon) zugeordnet.

### 3. Infektion/Notfallszenario:

a) Der Nutzer teilt dem Gesundheitsamt seine User ID und ggf. weitere Daten mit. Damit kann das Amt mit Hilfe der auf dem Server gespeicherten Daten feststellen, an welchen Venues der Nutzer z.B. in den letzten zwei Wochen gewesen ist. Hierzu kann ein Entitäts-Gerät des Amtes beispielsweise in Schritt 106 eine Mitteilung mit der User-ID dieses Nutzers an den Server senden;
b) Der Server erzeugt daraufhin in Schritt 108 auf Basis der User-ID und optional den weiteren Daten eine Vielzahl von Kandidaten-Trace-IDs;
c) Dann wird in Schritt 110 abgefragt, bei welchen Venues der zu tracende Nutzer in einem vergangenen Zeitraum einen Check-in vorgenommen hat. Hierfür wird eine Suche mit den Kandidaten-Trace-IDs in den Check-in-Datensätzen des Servers durchgeführt, um eine Liste der Venue-IDs der besuchen Venues zu erhalten;
d) In Schritt 112 werden die ermittelten Venue-IDs ausgegeben.
e) Dann wird in optionalen weiteren Schritten abgefragt, welche User-IDs gleichzeitig mit dem infizierten User in dem relevanten Zeitraum an den Venues waren.
f) Die verschlüsselten schutzbedürftigen Datensätze dieser User können dann mit dem privaten Entitätenschlüssel, z.B. Gesundheitsamtsschlüssel, entschlüsselt werden und vom Gesundheitsamt kontaktiert werden. Dies kann z.B. wie folgt erfolgen: es werden anhand der Venue-IDs, Check-in Zeiten und optional vorhandenen Check-Out-Zeiten, die alle jeweils im Klartext in einem Check-in Datensatz enthalten sind, alle Check-in Datensätze ermittelt, die zu Check-in Vorgängen gehören, die in zeitlicher Überlappung mit den Check-ins des zu tracenden Nutzers an den gleichen Venues erfolgt sind. Die so ermittelten Check-in daten der Kontaktpersonen (und möglichen Infizierten) enthalten beispielsweise verschlüsselte Ausweisdaten der jeweiligen Kontaktpersonen, die mit dem öffentlichen Entitätenschlüssel des Amts verschlüsselt sind, also vom Amt entschlüsselt werden können. Die entschlüsselten Ausweisdaten der Kontaktpersonen wiederum enthalten den Nutzerdaten-Entschlüsselungsschlüssel, mittels welchen das Amt die Nutzerdaten der Kontaktpersonen entschlüsseln kann, um die Kontaktpersonen darüber zu informieren, dass sie mit einem Infizierten für eine gewisse Zeit die gleiche Venue besucht haben.

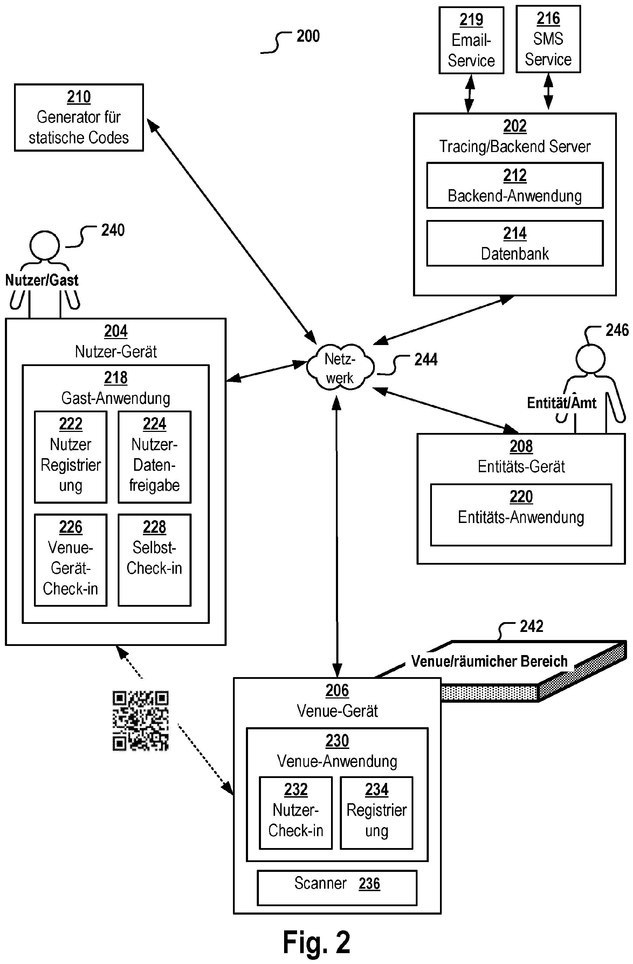

**Figur 2** zeigt ein Blockdiagramm eines verteilten elektronischen Systems 200 zum Tracing von Nutzern.

Das System 200 umfasst einen Server 202, welcher zum Beispiel von einem Betreiber eines Tracing-Dienstes bereitgestellt wird. Der Server kann eine Backend-Anwendung 212 umfassen, die verschiedene Funktionalitäten umfassen kann, zum Beispiel eine Funktionalität zur Registrierung von Nutzern 240, Venues 242, und Entitäten 246, und/oder zur Speicherung, Verwaltung und Durchsuchung von Nutzer- und Check-in Daten und/oder zur Erzeugung von Kandidaten-Trace-IDs. Zur Speicherung der Daten, insbesondere von Kontaktdaten der bei dem Server 202 registrierten Nutzer, Venues und Entitäten, zur Speicherung von Check-in-Datensätzen und/oder Tracing-Datensätzen kann der Backend Server eine Datenbank 214 beinhalten oder operativ an diese gekoppelt sein. Bei der Datenbank kann es sich zum Beispiel um eine relationale Datenbank oder eine andere Form von strukturierter Datenablage handeln.

In manchen Beispielen kann der Server einen E-Mail Service 218 und/oder einen SMS Service 216 umfassen, die dazu genutzt werden können, die bei dem System registrierten Nutzer im Bedarfsfall zu kontaktieren und beispielsweise zu warnen, dass sie sich gleichzeitig mit einer infizierten Person in einem bestimmten Venue aufgehalten haben. Der SMS-Service kann zur Validierung von Telefonnummern verwendet werden.

Das System umfasst gemäß Ausführungsformen der Erfindung ferner mehrere Nutzergeräte 204, die jeweils einem Nutzer 240 zugewiesen sind. Bei den Nutzergeräten handelt es sich um Datenverarbeitungsgeräte, insbesondere um portable Datenverarbeitungsgeräte, zum Beispiel Mobilfunkgeräte. Bei den Nutzern, denen die Nutzergeräte jeweils zugeordnet sind, handelt es sich um Personen, die sich temporär in verschiedenen räumlichen Bereichen (Venues) aufhalten können und somit als (zahlende oder nicht zahlende) Gäste des Venues fungieren können. Auf jedem der Nutzergerät kann ein Anwendungsprogramm 214, hier als Gast-Anwendung bezeichnet, instanziiert sein, welches mit dem Backend-Anwendungsprogramm 212 des Servers 202 interoperabel ist. Das Anwendungsprogramm 218 kann zum Beispiel als App ausgebildet sein und verschiedene Funktionen umfassen. Zu den Funktionen können zum Beispiel eine Funktion 222 zur Registrierung des Nutzers gegenüber dem Server 202, eine Funktion 226 zum Check-in des Nutzers 240 gegenüber einem Venue, eine Funktion 228 zum Selbst-Check-in gegenüber einem Venue, der anstatt über ein Venue-Gerät nur über ein Trägerobjekt mit einer automatisch erfassbaren Venue-ID ausgestattet ist, und/oder einer Funktionalität 224 zur Übermittlung eines Tracinggeheimnis-Transferobjekts mit nutzerbezogenen sensiblen Daten, um einer Entität 246 Zugriff auf die Nutzerdaten des Nutzers zu gewähren, gehören.

Gemäß Ausführungsformen der Erfindung umfasst das System ferner mehrere Entitäts-Geräte 208, die jeweils einer Entität 246 zugewiesen sind. Bei der Entität handelt es sich um eine vertrauenswürdige, im allgemeinen hoheitliche Instanz, zum Beispiel ein Amt oder eine Behörde, zum Beispiel ein Gesundheitsamt. Damit die Entität das Tracing-System nutzen kann, muss es sich gegenüber dem Server 202 registriert haben, wobei im Zuge der Registrierung der Entität ein asymmetrisches kryptographisches Entität-Schlüsselpaar erzeugt wird, das gemäß Ausführungsformen der Erfindung regelmäßig, zum Beispiel täglich, durch ein aktuell gültiges Schlüsselpaar ersetzt wird. Der öffentliche Teil dieses Schlüsselpaares wird an den Server 202 bereitgestellt, um diesem zu ermöglichen, den öffentlichen Verschlüsselungsschlüssel der Entität an die Nutzergeräte 204 und/oder die Venue-Geräte 206 zur Verfügung zu stellen, sodass diese mithilfe des öffentlichen Entitäten-schlüssels bestimmte Teile der Check-in Datensätze (insb. die Ausweis-Datensätze) und optional auch die Nutzerdaten verschlüsseln können, bevor die besagten Daten zum Server hochgeladen und dort zentral in der Datenbank 214 gespeichert werden. Es ist möglich, dass sich mehrere verschiedene Entitäten (zum Beispiel Gesundheitsbehörde) bei dem Server registriert haben und dass zu jeder Entität mehrere Entität-Geräte gehören, zum Beispiel Datenverarbeitungsgeräte (Computer) der Mitarbeiter der jeweiligen Behörde. Nach Ausführungsformen der Erfindung können mehrere Entitäten dadurch in das System integriert werden, dass sie das gleiche asymmetrische Entitäten-Schlüsselpaar zur Ver- und Entschlüsselung von Check-in-Daten und/oder Nutzerdaten verwenden, was zum Beispiel dadurch realisiert sein kann, dass die Entitäts-Anwendung eine Funktionalität zur Synchronisation sowohl des öffentlichen als auch des privaten Anteils dieses asymmetrischen kryptographischen Schlüsselpaares mit allen anderen Entitäts-Anwendungen der gleichen oder einer kooperierenden Entität (oder aller Entitäten, die funktional als eine Entität fungieren sollen) beinhaltet. Vorzugsweise werden zumindest die privaten Teile des Schlüsselpaars in verschlüsselter Form ausgetauscht und synchronisiert, zum Beispiel indem sie mit einem öffentlichen Transportschlüssel des jeweiligen Empfänger-Entität-Gerät verschlüsselt werden.

Gemäß einer Ausführungsform wird die Synchronisation der Entitäten-Schlüssel zwischen mehreren Mitarbeitern und Entitäten-Geräten einer Entität durch tägliche Schlüsselrotation realisiert: Die Schlüssel werden durch eine Key-ID (Ganzzahl) identifiziert, die mit jedem neuen Schlüssel inkrementiert wird. Die Rotation wird von jedem Mitarbeiter des Gesundheitsamtes ausgelöst, der sich nach Ablauf der Gültigkeit des letzten für z.B. einen Tag gültigen öffentlichen Entitäten-Schlüssels einloggt. Der tägliche öffentliche Entitäten-Schlüssel ist das alleinige Eigentum der Gesundheitsbehörden und wird verwendet, um die Check-in Daten des Nutzers zu sichern.

Das Rotationsverfahren beinhaltet, dass im Zuge der Registrierung und/oder im Zuge eines Check-ins eines Nutzers der jeweils aktuell gültige öffentliche Tagesschlüssel der Entität an das Nutzergerät übertragen wird, sodass das Nutzergerät den Schlüssel zur Verschlüsselung der Ausweisdaten bei dem Check-in und optional auch der Nutzerdaten bei der Nutzerregistrierung verwenden kann. Die Ablaufzeit eines täglichen Schlüsselpaares beträgt z.B. 24 Stunden. Wenn ein tageweise gültiges Entitätenschlüsselpaar abgelaufen ist, generiert der Mitarbeiter des Amts ein neues für den aktuellen Tag gültiges Entitätenschlüsselpaar, dessen öffentlicher Schlüssel von der Entitäten-Applikation zum Server hochgeladen wird und vom Server über das Netzwerk automatisch an die Nutzergeräte der Nutzer verteilt wird. Außerdem laden sich die teilnehmenden Entitäten-Geräte die jeweiligen (statischen) öffentlichen Schlüssel der anderen Entitäten-Geräte der Entität herunter. Das Entitäten-Gerät, das eben das tagesaktuelle Entitäten-Schlüsselpaar erzeugt hat, verschlüsselt mit den öffentlichen statischen Schlüsslen der jeweils anderen Entität-Geräte den aktuellen privaten tageweise gültigen EntitätenSchlüssel, und sendet dieses so gebildete Chiffrat an das betreffende Entitäten-Gerät, das das Chiffrat entschlüsselt und somit ebenfalls im Besitz des tageweise gültigen privaten Entitäten-Schlüssels ist. Es ist auch möglich, dass verschiedene Gesundheitsämter durch das besagte Schlüsselrotationsverfahren sich täglich (oder in einem anderen vordefinierten Zeitintervall) neue private Entitätenschlüssel zusenden.

Dieser Prozess geschieht vorzugsweise vollautomatisch im Hintergrund ohne aktives Zutun der Mitarbeiter des Amts und/oder der einzelnen Nutzer.

Gemäß Ausführungsformen der Erfindung umfasst das System ferner mehrere Venue-Geräte 206 mehrerer verschiedener Venues 242. Es ist möglich, dass an einem Venue mehrere Venue-Geräte 206 angebracht sind, zum Beispiel jeweils ein Gerät an jedem von mehreren Eingängen zu dem Venue. Auf jedem Venue-Gerät kann eine Venue Anwendung 230 instanziiert sein, die zum Beispiel Funktionalitäten 232, 234 für einen Check-in Vorgang eines Nutzers gegenüber dem Venue und/oder für die Registrierung des Venues gegenüber dem Server 202 beinhaltet. Beispielsweise kann das Venue-Gerät 206 eine Kamera beinhalten, die dazu ausgebildet ist, einen QR-Code, der auf einem Display des Nutzergeräts 204 angezeigt ist und in welchem Check-in Daten eines Gastes kodiert sind, zu erfassen. Der QR-Code wird von der Venue-Anwendung 230 dekodiert und zumindest einige der dekorierten Daten werden verknüpft mit einer Venue-ID des Venues 242 auf dem Server 204 gespeichert.

Optional kann das System ein oder mehrere weitere Datenverarbeitungsgeräte 210 für die Erzeugung statischer graphischer Codes beinhalten. Dies kann vorteilhaft sein, da hierdurch auch Personen das Tracing-System 200 nutzen können, die nicht sehr IT-affin sind und keinen eigenen Nutzeraccount anlegen können oder wollen. Beispielsweise kann ein Mitarbeiter einer Pflegeeinrichtung sich oder die Pflegeeinrichtung als Nutzer gegenüber dem Server 202 registrieren und das Datenverarbeitungsgerät 210 dazu veranlassen, eine bestimmte Menge, zum Beispiel 50 oder 100 Stück, eines QR-Codes zu erstellen. Diese vorab erstellten und noch nicht personalisiert QR-Codes können dazu verwendet werden, an die Gäste des Pflegeheimes verteilt zu werden, um diesen zu ermöglichen, ein Venue zu besuchen (zum Beispiel einen Konzertraum, ein Theater, etc.), ohne hierfür einen eigenen Account anlegen zu müssen. Die Personen, die die QR-Codes bestellt und zum Beispiel per Post oder über das Internet empfangen und ausgedruckt hat, verteilt die QR-Codes an die Gäste des Pflegeheimes und notiert im Zuge der Ausgabe eine Zuweisung des ausgegebenen QR-Codes an eine konkrete Person.

Beispielsweise können die bestellten QR-Codes mit einer Seriennummer versehen sein, und das Pflegepersonal erstellt handschriftlich oder elektronisch eine Liste, in welcher eine Zuweisung des ausgegebenen Codes an diejenige Person enthalten ist, die den Code erhalten hat. Die Liste kann zum Beispiel auf Papier erstellt werden, sie kann aber auch über eine Funktionalität eine Anwendung auf dem Generatorgerät 214, die zum Beispiel über das Internet bereitgestellt wird, erstellt und gespeichert werden.

Die einzelnen Komponenten des Systems sind über Netzwerk 244 miteinander verbunden. Bei dem Netzwerk kann es sich zum Beispiel um das Internet handeln, wobei Daten kabelgebunden und/oder drahtlos übermittelt werden kann. Beispielsweise kann die Datenübertragung zwischen Nutzergeräten 204 und Server 202 über eine Mobilfunkverbindung oder WLAN erfolgen. Die Übertragung von Check-in Daten von dem Nutzergerät eines Nutzers an ein Venue Gerät 206 kann beispielsweise über einen optischen Datenübertragungskanal und/oder über ein Nahfeldsignal wie zum Beispiel Bluetooth erfolgen.

Untenstehende Tabelle zeigt verschiedene Verfahrensschritte gemäß einer Ausführungsform der Erfindung, die von verschiedenen Systemkomponenten ausgeführt werden, sowie die jeweiligen Input und Output Daten.

| **Schritte** | **Ziel** | **Inputdaten** | **Outputdaten** |
|---|---|---|---|
| Venue Registrierung (bei dem Server) | Ermöglicht es privaten und gewerblichen Venue-Betreibern, Gäste automatisch zu registrieren und im Notfall rückwirkend zu tracen. | E-Mail, Passwort, Standortinformationen | Venuespezifisches asym. Schlüsselpaar venuePrivKey und VenuePubKey |
| Entitäten Registrierung (bei dem Server) | Ermöglicht es Angestellten eines Amtes, Besucher der Venues zu tracen | Zertifikat | Entitätsspezifisches asym. Schlüsselpaar: healthPrivKey healthPubKey |
| Nutzer Registrierung (bei dem Server) | Erlaubt es dem Nutzer, mittels des Systems automatisch einzuchecken bzw. auszuchecken | Nutzerdaten, insb. Adresse und Kontaktdaten | anonymisierter QR Code, userPrivKey user-PubKey, mehrere Geheimnisse |
| Check-in/out eines Nutzers bei einer Venue | Übermittlung anonymer Kontaktinformation an den Venue-Betreiber Scanner check-in Selbst-Check-in mittels QR Code auf Trägerobjekt | anonymer QR Code | Hochgeladene Check-in Daten |
| Kontakt-Tracing (Notfall/Infektion) | Übermittlung von Nutzerkontaktdaten ans Gesundheitsamt Aufdeckung möglicher Kontaktpersonen durch das Gesundheitsamt, optional nur nach Freigabe durch Venue | TAN des infizierten Nutzers, privater Entschlüsselungsschlüssel des Gesundheitsamts healthPrivKey | Vom Infizierten besuchte Venues, Kontaktdaten möglicher Kontaktpersonen |

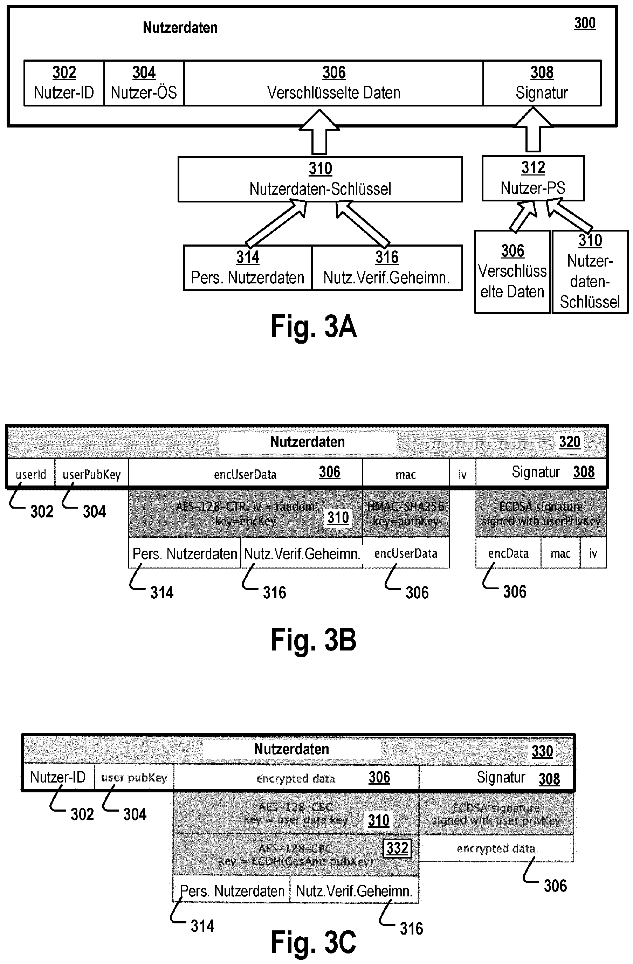

**Figuren 3A-3C** zeigen verschiedene Beispiele für die Erzeugung und Speicherung von Nutzerdaten ("User-Data-Package", "Nutzerdaten-Datensatz").

**Figur 3A** zeigt einen Nutzerdaten-Datensatz 300, wie er gemäß einer Ausführungsform auf dem Server 202 gespeichert ist. Der Datensatz 300 umfasst zumindest eine Nutzer-ID 302 im Klartext, mit welcher weitere nutzerbezogene Daten verknüpft auf dem Server gespeichert sind. Zu diesen weiteren Daten gehören zumindest verschlüsselte Nutzerdaten 306 einer Person. Optional können weitere personenbezogene Daten in dem Nutzerdaten-Datensatz 300 enthalten sein, zum Beispiel ein öffentlicher Schlüssel 304 eines asymmetrischen kryptographischen Schlüsselpaares, welches spezifisch für diesen Nutzer im Zuge dessen Registrierung ausgestellt wurde. Optional können die Nutzerdaten weitere Daten wie zum Beispiel eine Signatur 308 enthalten.

Im unteren Teil der Figur 3A ist dargestellt, wie verschiedene Teile der Nutzerdaten 300 erzeugt werden können. Beispielsweise können die verschlüsselten Daten 306 dadurch erzeugt werden, dass die persönliche Nutzerdaten 314 des Nutzers, also zum Beispiel der Name, die Adresse und/oder sonstige Kontaktinformationen des Nutzers, mit einem Nutzerdaten-Verschlüsselungsschlüssel 310 verschlüsselt werden. Beispielsweise kann es sich bei dem Schlüssel 310 um einen symmetrischen kryptographischen Schlüssel handeln, der im Zuge der Registrierung des Nutzers spezifisch für diesen Nutzer erzeugt wurde und welcher gleichzeitig auch eine Entschlüsselung der verschlüsselten Daten 306 ermöglicht. Vorzugsweise werden die persönlichen Nutzerdaten 314 zusammen mit einem nutzerspezifischen Verifikationsgeheimnis 316 von dem Nutzerdaten-Schlüssel 310 verschlüsselt und/oder das Geheimnis 316 wird als Parameter im Zuge der Verschlüsselung zur Erzeugung der verschlüsselten Nutzerdaten 306 verwendet.

In einer alternativen Ausführungsform wird im Zuge der Registrierung des Nutzers der UserDataKey als asymmetrisches kryptographisches Schlüsselpaar erzeugt. In diesem Fall muss nur der zur Entschlüsselung verwendete private Teil geschützt im Nutzergerät gespeichert und als Bestandteil der Ausweisdaten bzw. Check-in Daten beim Check-in in verschlüsselter Form an den Server übermittelt werden.

Die optionale Signatur 308 kann beispielsweise dadurch erzeugt werden, dass ein privater kryptographischer Schlüssel (Signaturschlüssel) 312, der spezifisch für den Nutzer bei dessen Registrierung erzeugt wurde, die verschlüsselten Nutzerdaten 306 und den zur Verschlüsselung der Nutzerdaten 314 verwendeten Nutzerdatenschlüssel 310 signiert. Bei dem öffentlichen Schlüssel 304 kann es sich um einen zu dem Schlüssel 312 korrespondierenden Signaturprüfschlüssel handeln. Dies erlaubt es dem Amt, zu prüfen, ob der Nutzerdatensatz nach dessen Speicherung und Signierung eventuell nachträglich manipuliert wurde.

In **Figur 3B** ist ein Nutzerdaten-Datensatz 320 gemäß einer weiteren Ausführungsform dargestellt. Datenwerte bzw. Schlüssel des Datensatzes 320, die einem der Datenwerte bzw. Schlüssel des Datensatzes 300 inhaltlich und/oder funktional entsprechen, sind mit den gleichen Referenznummern gekennzeichnet. Die mit dem Rahmen umgrenzte Box kennzeichnet den Nutzerdatensatz, wie er auf dem Server gespeichert ist. Die darunter stehenden Boxen repräsentieren Inputdatenwerte bzw. Schlüssel, die zur Erzeugung entsprechender Elemente des Nutzerdatensatzes verwendet wurden. Bei dem mac handelt es sich um einen Message Authentication Code, der z.B. mittels eines HMAC-SHA256 Algorithmus auf Basis der verschlüsselten Nutzerdaten 306 erstellt wurde. Das Kürzel "iv" repräsentiert einen Zufallswert. Die Signatur umfasst in diesem Fall nicht nur die verschlüsselten Nutzerdaten 306, sondern auch den mac und den Zufallswert iv.

In **Figur 3C** ist ein Nutzerdaten-Datensatz 330 gemäß einer weiteren Ausführungsform dargestellt. Datenwerte bzw. Schlüssel des Datensatzes 330, die einem der Datenwerte bzw. Schlüssel des Datensatzes 300 inhaltlich und/oder funktional entsprechen, sind mit den gleichen Referenznummern gekennzeichnet. Die mit dem Rahmen umgrenzte Box kennzeichnet den Nutzerdatensatz, wie er auf dem Server gespeichert ist. In der hier gezeigten Ausführungsform werden die verschlüsselten Nutzerdaten 306 durch eine zweifache Verschlüsselung gebildet. In einem ersten Verschlüsselungsschritt werden die persönlichen Nutzerdaten 314 sowie vorzugsweise auch ein Nutzerverifikationsgeheimnis 316 mit einem öffentlichen Entitäten-Schlüssel 332 verschlüsselt. Das in diesem Schritt erzeugte Chiffrat wird in einem weiteren Verschlüsselungsschritt mit dem nutzerspezifisch erstellten User-Data-Key 310 erneut verschlüsselt, um die (doppelt) verschlüsselten Nutzerdaten 306 zu erhalten und auf dem Server zu speichern.

Gemäß einer Ausführungsform stellt der Benutzer der App (Anwendungsprogramm) 218 seines Nutzergeräts seine Kontaktinformationen 314 im Zuge eines Registrierungsprozesses zur Verfügung. Die Anwendung erzeugt ein EC secp256r1-Schlüsselpaar (user public key und user private key) 312, 304, ein Benutzer-Trace-Geheimnis 410, d.h. ein user trace secret (16 Bytes Entropie), ein Benutzer-Verifizierungs-Geheimnis 412, d.h. user verification secret (16 Bytes Entropie) und einen symmetrischen Benutzer-Datenschlüssel, d.h. user data key (16 Bytes Entropie) 310.

Der Nutzerdaten-Schlüssel 310 wird zur Verschlüsselung der persönlichen Nutzerdaten 314 verwendet, um die verschlüsselten Nutzerdaten 306 zu erhalten.

In manchen Ausführungsformen in welchen zusätzlich eine Verschlüsselung durch einen Entitäten-Schlüssel erfolgt, kann die Anwendung einen Verschlüsselungsschlüssel mit ECDH mit dem privaten Schlüssel des Benutzers und dem öffentlichen Hauptschlüssel (Entitätenschlüssel) des Gesundheitsamtes berechnen. Die App 218 berechnet ein HMAC-SHA256 mit der ECDH-Ausgabe als Daten und dem Datenschlüssel als Schlüssel. Die Anwendung verschlüsselt dann die persönlichen Informationen und das Benutzerverifizierungsgeheimnis mit diesem Verschlüsselungsschlüssel, zum Beispiel mit AES-128-CBC.

Die Anwendung 218 lädt dann diese verschlüsselten Daten auf den Server 202 und signiert sie mit dem privaten Schlüssel 312 des Benutzers zusammen mit dem öffentlichen Schlüssel des Benutzers. Der Server gibt eine Benutzerkennung, d.h. User-ID, zurück.

Im Nutzergerät werden gespeichert:
- user id (uuid) 302,
- user public key (ec secp256r1) 304,
- user private key (ec secp256r1) 312,
- user data key (16 bytes random data) 310,
- user trace secret (16 bytes random data) 410,
- user verification secret (16 bytes random data) 412.

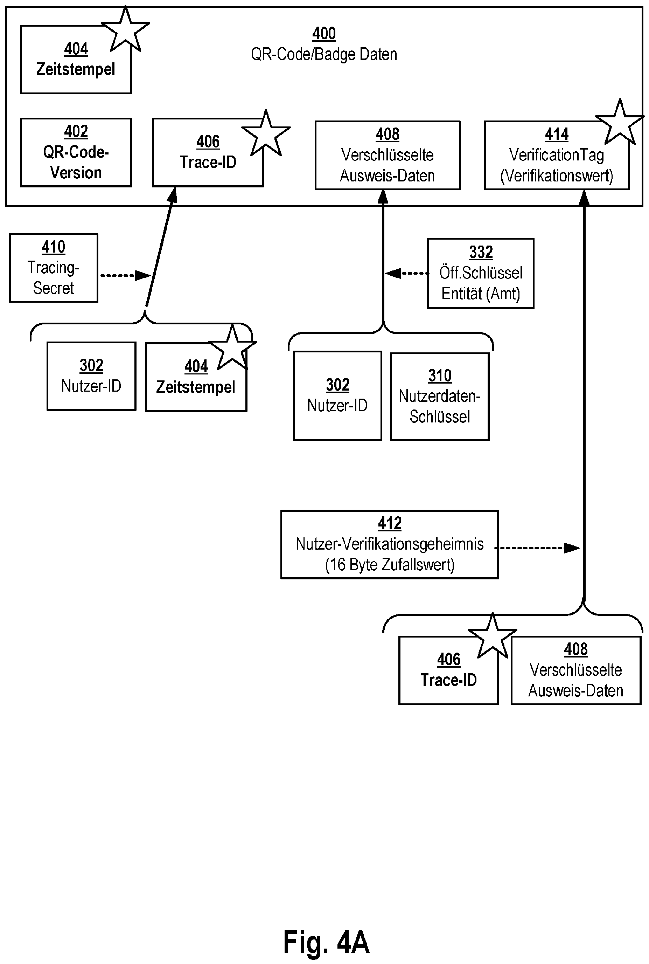

**Figuren 4A-3C** zeigen verschiedene Beispiele 400, 420, 430 für den Inhalt eines Check-in QR-Codes. Falls nicht ein graphischer Code zur Übermittlung der Check-in-Daten eines Nutzers an das Venue-Gerät verwendet wird, sondern zum Beispiel ein Nahfeld-Signal, sind die bezeichneten Daten nicht in Form eines graphischen Codes, sondern als Nahfeldsignal, zum Beispiel Bluetooth Signal, repräsentiert.

Beispielsweise kann das Nutzergerät eine eigene Uhr, insbesondere eine Echtzeituhr, beinhalten und/oder eine aktuelle Uhrzeit regelmäßig über das Netzwerk von einem Uhrzeit-Server empfangen.

Wenn der Nutzer Vorkehrungen zur Durchführung eines Check-in gegenüber einem Venue durchführt, also zum Beispiel eine App startet oder ein entsprechendes GUI-Element der App bedient, zum Beispiel um einen graphischen Check-in Code zu erzeugen, erfasst das Nutzergerät einen aktuellen Zeitwert 404 und führt eine Ableitfunktion auf diesem Zeitwert 404 und der Nutzer-ID 302 aus, um eine Trace-ID 406 zu erhalten. Vorzugsweise geht diese Ableitung ein Tracing-Secret 410 ein, dass zum Beispiel ein Zufallswert sein kann, der dem Nutzer im Zuge der Registrierung zugewiesen und geschützt auf dem Nutzergerät gespeichert ist. Vorzugsweise stellt die Art der Ableitfunktion sicher, dass aus der Trace-ID die Nutzer-ID nicht im Klartext hervorgeht und vorzugsweise es auch nicht anhand der Trace-IDs ersichtlich ist, ob sie demselben oder unterschiedlichen Nutzern angehören. Der Umstand, dass eine aktuelle Zeitinformation 404 in die Trace-ID 406 eingeht, ist durch den Stern symbolisiert.

In analoger Weise können sich auch Venues, repräsentiert jeweils durch ein oder mehrere Venue-Geräte, bei dem Server registrieren. Zu den im Zuge der Registrierung hinterlegten Daten können u.a. gehören: Name des Standortes, Name und E-Mail der Kontaktperson, Adresse des Standortes, Koordinaten des Standortes und/oder Eincheck-Radius.

Der Datensatz 400 wie in **Figur 4A** gezeigt enthält außerdem verschlüsselte Ausweisdaten 408, die dadurch gebildet werden, dass mit einem öffentlichen Verschlüsselungsschlüssel 332 der Entität die Nutzer-ID 302 des Nutzers und der nutzerspezifische Entschlüsselungsschlüssel 310 (zum Beispiel der symmetrische User-Data-Key) verschlüsselt werden. Durch die Ausweisdaten 408, in welchen die User-ID verschlüsselt enthalten ist, ist also eine Zuweisung dieses Check-in Datensatzes an einen konkreten Nutzer möglich, allerdings nur für eine Partei, die die Ausweisdaten 408 entschlüsseln kann (also die Entität). Da die Ausweisdaten aber nur die Nutzer-ID und nicht die gesamten Nutzerdaten 314 enthalten, sind diese so klein, dass sie sich für die Kodierung innerhalb eines QR-Codes eignen. Wenn das Amt mit seinem privaten Entitätenschlüssel die verschlüsselten Ausweisdaten 408 entschlüsselt, erfährt es nicht nur die User-ID des Nutzers, zu welchen dieser Check-in Datensatz 400 gehört, sondern erhält auch den Entschlüsselungsschlüssel 310, der notwendig ist, um die Kontaktdaten des Nutzers in Erfahrung zu bringen.

Optional kann der QR-Code-Datensatz 400 auch einen Verifikationsdatenwert 414 enthalten, welcher einen zusätzlichen Schutz bietet, da er der Entität eine Prüfung erlaubt, ob der Check-in Datensatz manipuliert wurde.

Gemäß einer Ausführungsform wird der Verifikationsdatenwert 414 dadurch erzeugt, dass die Trace-ID 406 und die verschlüsselten Ausweisdaten 408 mit einem Nutzer-Verifikationsgeheimnis 412 verschlüsselt werden. Bei dem Nutzer-Verifikationsgeheimnis handelt es sich vorzugsweise um eine Zufallszahl, die im Zuge der Registrierung des Nutzers bei dem Server erzeugt wird und auf einem Speicher des Nutzergerätes des Nutzers geschützt gespeichert wird. Falls ein unberechtigter Nutzer also versuchen sollte, eine Trace-ID eines anderen Nutzers zu stehlen und einen manipulierten Check-in Datensatz mit der gestohlenen Trace-ID zu verwenden, so wäre es dem unberechtigten Nutzer nicht möglich, den zu dieser Trace-ID passenden Verifikationsdatenwert 414 zu erzeugen, solange sich der unberechtigte Nutzer nicht auch noch in den Besitz des Nutzergeräts des berechtigten Nutzers begibt, da nur dort das Nutzer-Verifikationsgeheimnis 412 gespeichert ist, das zur Ableitung des Verifikationsdatenwertes 414 erforderlich ist.

Das Nutzergerät ist dazu konfiguriert, regelmäßig zu prüfen, ob die aktuell erzeugten Check-in Daten noch aktuell sind. Falls die Trace-ID Ableitfunktion vorsieht, immer die aktuelle Zeit abgerundet auf die Minute als Zeitstempel 404 zur Ableitung der Trace-ID 406 zu verwenden, gilt ein QR-Code bzw. Check-in Datensatz 400 als veraltet, sobald die neue Minute begonnen hat. In diesem Fall verwirft das Nutzergerät automatisch den aktuellen Check-in Datensatz 400 bzw. den QR-Code 400 und erzeugt wie oben beschrieben einen neuen, aktuellen Check-in Datensatz auf Basis der aktuellen Zeitinformation 404, also auf Basis zum Beispiel der neu begonnenen abgerundeten Minute.

Falls dem Venue kein Venue-Gerät zugewiesen ist, sondern lediglich ein Trägerobjekt, auf oder an welchem die Venue-ID so angebracht ist, dass das Nutzergerät diese automatisch erfassen kann, kann die Erzeugung des Check-in Datensatzes 400 vom Nutzergerät ausgeführt werden, ohne dass dieser an ein Venue-Gerät übermittelt wird und ohne dass dieser Angaben zu einer QR-Code Version oder sonstige technische Parameter zur Datenübertragung an ein Venue-Gerät enthält. Vielmehr kann ein solcher Datensatz dann direkt mit der vom Nutzergerät empfangenen Venue-ID angereichert und als fertiger Check-in-Datensatz an den Server übermittelt werden.

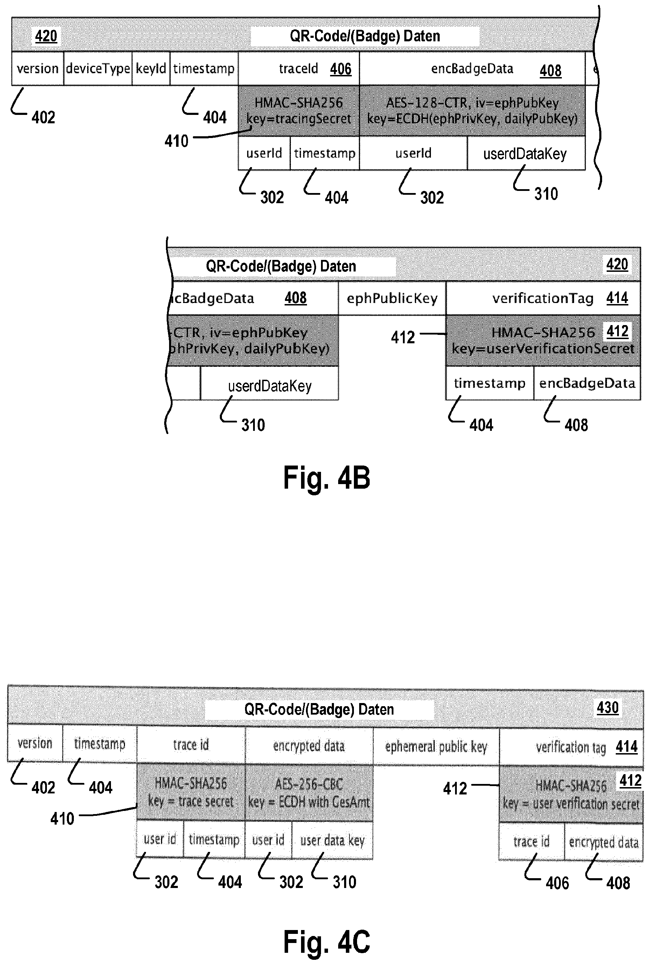

In **Figur 4B** ist ein Check-in-Datensatz 420 bzw. ein QR-Code, der diesen kodiert, gemäß einer weiteren Ausführungsform dargestellt. Datenwerte bzw. Schlüssel des Datensatzes 420, die einem der Datenwerte bzw. Schlüssel des Datensatzes 400 inhaltlich und/oder funktional entsprechen, sind mit den gleichen Referenznummern gekennzeichnet. Die ersten beiden Zeilen der Datenstruktur der Figur 4B zeigen den Teil des Check-in Datensatzes 420, wie er (bei den Check-in Varianten mit Venue-Gerät) an das Venue-Gerät übermittelt wird. Die darunter stehenden Boxen repräsentieren Inputdatenwerte bzw. Schlüssel, die zur Erzeugung entsprechender Elemente des Datensatzes 420 verwendet wurden. Die Datenstruktur 420 enthält noch weitere optionale Datenwerte wie zum Beispiel die Version 402 des zur Erzeugung des QR-Codes verwendeten Algorithmus ("QR-Code Version"), den Gerätetyp des Nutzergerätes, einen Identifikator des (täglich aktualisierten) asymmetrischen kryptographischen Entitäten-Schlüsselpaares, dessen öffentlicher Teil zur Verschlüsselung der Ausweis-Daten verwendet wurden, etc. Zusätzlich oder alternativ zu der im Klartext vorhandenen Check-in Zeit 404 kann auch eine Check-out Zeit enthalten sein.

In **Figur 4C** ist ein Check-in-Datensatz 430 bzw. ein QR-Code, der diesen kodiert, gemäß einer weiteren Ausführungsform dargestellt. Im Unterschied zu den Datenstrukturen 400 und 420 ist bei der Datenstruktur 430 der Nutzerverifikationsdatenwert 414 von der aktuellen Zeit indirekt (über die Trace-ID) abgeleitet.

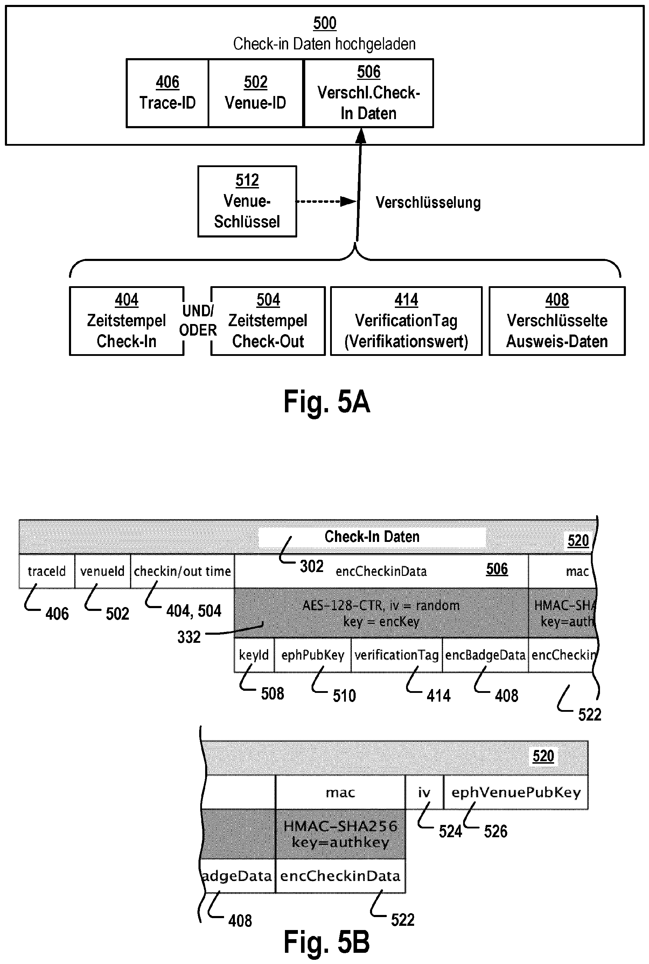

**Figuren 5A-5C** zeigen verschiedene Beispiele für die Erzeugung und Speicherung von Check-in Daten, wie sie auf dem Server gespeichert werden.

Im Zuge des Check-Prozesses empfängt das Venue-Gerät einen Check-in Datensatz 400, 420, 430 von dem Nutzergerät des Nutzers. Die in dem empfangenen Datensatz enthaltenen Daten können, wie sie sind, mit weiteren Daten, insbesondere der Venue-ID, verknüpft werden, um einen kompletten Check-in Datensatz zu erhalten, der auf dem Server gespeichert wird. Somit können die Check-in Datensätze des Servers aussehen wie die Datensätze 400, 420, 430, jeweils verknüpft mit weiteren Daten, insbesondere der Venue-ID des Venues des Venue-Gerätes.

Zumindest einige der Check-in Datensätze können faktisch Check-out Datensätze sein, und zusätzlich oder alternativ zu der Check-in Zeit 404 die Check-out Zeit 504 beinhalten.

In manchen Ausführungsformen führt das Venue-Gerät weitere Datenverarbeitungsschritte mit den vom Nutzergerät erhaltenen Check-in Daten 400, 420, 430 durch, um auf Basis der von dem Nutzergerät empfangenen Check-in Datensätze die fertigen, an den Server zu übermittelnden Check-in Datensätze zu erzeugen.

Beispielsweise kann jedem Venue im Zuge der Registrierung ein symmetrisches oder asymmetrisches Schlüsselpaar zugewiesen werden und das Venue-Gerät kann den privaten Teil des Schlüsselpaares (im asymmetrischen Fall) dazu verwenden, zumindest Teile jedes Check-in Datensatzes (abgesehen von der Trace-ID, die im Klartext vorliegenden muss), zu verschlüsseln. Manche Teile des Check-in Datensatzes liegen also in zweifach oder gar dreifach verschlüsselter Form vor: so sind zum Beispiel die Ausweisdaten 408 mit dem öffentlichen Schlüssel 332 der Entität erzeugt worden, sie können optional zusätzlich mit dem u-ser-Data-Key 310 des Nutzers verschlüsselt sein, und optional außerdem mit dem öffentlichen Venue-Schlüssel. Dies kann den Vorteil haben, dass die Entität ein Tracing von Nutzern, die einen Venue besucht haben, nur mit Zustimmung des Betreibers des Venues vornehmen kann, da der Betreiber "seine" Check-in Datensätze zunächst entschlüsseln muss, damit ein Entitäten-Gerät diese weiterverarbeiten kann. Neben dem Ausweisdaten-Chiffrat 408 kann das Venue-Gerät beispielsweise auch den Zeitstempel 404, den Nutzerverifikationsdatenwert 414 und weitere Daten mit dem privaten Venue-Schlüssel 512 verschlüsseln, um die verschlüsselten Check-in Daten 506 zu erhalten, die auf den Server 202 hochgeladen werden.

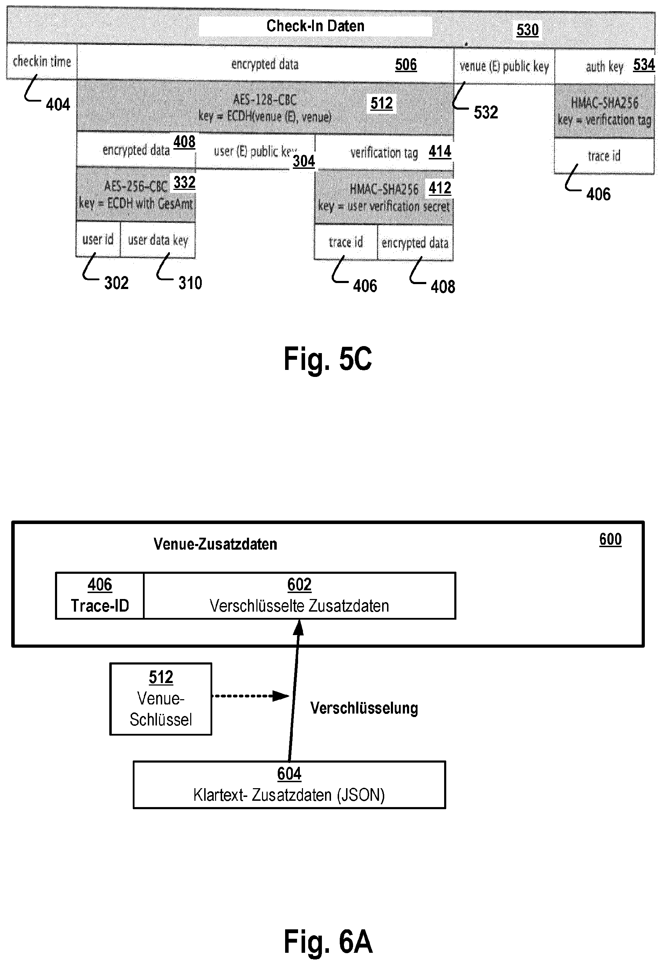

Die **Figuren 5B** **und** **5C** zeigen weitere alternative Varianten für Check-in Datensätze, wie sie auf dem Server gespeichert sind (erste beiden Zeilen) sowie darunterstehend die Datenwerte und Schlüssel, die zur Bildung der auf dem Server gespeicherten Werte verwendet werden.

Der Veranstaltungsort, d.h. das Venue-Gerät, prüft gemäß mancher Ausführungsformen zunächst nach Empfang von Check-in Daten, ob der Zeitstempel 404 neueren Datums ist und führt keine weitere Validierung durch.

Gemäß weiteren Beispielen können die verschlüsselten Check-in Daten 506 dadurch gebildet werden, dass die App 218 für jedes Einchecken ein Signal zur Übertragung der Trace-ID erzeugt, welches per direkter Kommunikation übertagen wird, beispielsweise per Anzeige eines QR-Codes, wobei auch ein ephemeres ECC-Schlüsselpaars secp256r1 und eines Verschlüsselungsschlüssels vorzugsweise für jeden neuen QR Code gebildet werden.

Die Trace-ID 406 kann z.B. ein HMAC-SHA256 sein, der aus der User-ID 302 und dem aktuellen Zeitstempel 404 (abgerundet auf die aktuelle Minute) als (Input)Daten und dem Benutzer-Trace-Geheimnis als Schlüssel abgeleitet und auf die ersten 16 Bytes gekürzt wird.

Der Verschlüsselungsschlüssel wird mit ECDH aus dem ephemeren privaten Schlüssel und dem öffentlichen Schlüssel des Gesundheitsamts berechnet. Die App wird einen QR-Code mit folgendem Inhalt anzeigen (der jede Minute mit dem aktuellen Zeitstempel aktualisiert wird):
- QR-Code-Version (1 Byte) 402,
- Zeitstempel (4 Bytes) 404,
- Trace-ID (16 Bytes) 406,
- verschlüsselte Daten (32 Bytes) 408, (Datenschlüssel und Benutzerkennung mit AES-128-CBC verschlüsselt und der Verschlüsselungsschlüssel als Schlüssel und die Trace-ID als IV) ephemerer öffentlicher Schlüssel (33 Bytes)
- Verifizierungs-Tag (8 Bytes) 414, (HMAC-SHA256 mit Trace-ID und verschlüsselten Daten als Daten und dem Verifizierungsgeheimnis des Benutzers als Schlüssel, abgeschnitten auf die ersten 8 Bytes).

Die Gesamtnutzlast beträgt 94 Bytes, die einfach verkettet und dann mit ASCII85 als QR-Code-Inhalt kodiert werden. Das ephemere Schlüsselpaar kann verworfen werden.

Der Veranstaltungsort verschlüsselt die vom Nutzergerät empfangenen verschlüsselten Daten, den ephemeren öffentlichen Schlüssel und den Verifizierungs-Tag z.B. mit AES-128-CBC mit dem ECDH eines ephemeren Schlüsselpaares und dem öffentlichen Schlüssel des Veranstaltungsortes. Der Veranstaltungsort erstellt einen Authentifizierungsschlüssel mit HMAC-SHA256 mit der Trace-ID 406 als Daten und dem Verifizierungsdatenwert ("Verifizierungstag") 414 als Schlüssel. Der Veranstaltungsort wird die Check-in-Zeit, die verschlüsselten Daten und den Authentifizierungsschlüssel auf den Server hochladen.

Wenn der Benutzer über die App (geofence oder manuell) auscheckt, sendet die App 218 gemäß Ausführungsformen der Erfindung einen Checkout-Zeitstempel 504 und einen HMAC-SHA256 mit dem Checkout-Zeitstempel als Daten und dem Authentifizierungsschlüssel (HMAC(data=trace id, key=verification tag) als Schlüssel).

Die Erzeugung der in den Figuren 4 und 5 gezeigten Datenstrukturen kann z.B. so erfolgen, dass die App 218 auf dem Nutzergerät eine Trace-ID für jeden neuen QR-Code generiert. Die Trace-ID ist ein HMAC-SHA256, abgeleitet von der User-ID und dem aktuellen Zeitstempel (abgerundet auf die aktuelle Minute) als Daten und dem Benutzer-Tracing-Geheimnis als Schlüssel, abgeschnitten auf die ersten 16 Bytes). Die Userld und der UserDataKey werden mit dem oben für den aktuellen DailyPublicKey beschriebenen asymmetrischen Verschlüsselungsschema verschlüsselt, wobei die IV in den Datensätzen 420 und 520 der Figuren 4B und 5B als die ersten 16 Bytes des ephemerenPublicKey definiert ist.

Die App 218 erzeugt gemäß eines weiteren Beispiels einen QR-Code mit folgendem Inhalt (der jede Minute mit dem aktuellen Zeitstempel aktualisiert wird) an (siehe Figur 4B und C): App Badge Data, QR-Code-Version (1 Byte), Gerätetyp (1 Byte), Schlüssel-ID (2 Byte) (gibt an, welcher DailyPublicKey verwendet wurde), Zeitstempel (4 Byte), Trace-ID (16 Bytes), verschlüsselte Daten (32 Bytes) (userDataKey und Benutzerkennung verschlüsselt nach dem oben beschriebenen asymmetrischen Schema für den öffentlichen Entitätenschlüssel mit den ersten 16 Bytes des ephemeren publicKey als IV), ephemerer öffentlicher Schlüssel (33 Bytes), sowie Verifizierungs-Tag (8 Bytes) (HMAC-SHA256 mit Zeitstempel und verschlüsselten Daten als Daten und dem Verifizierungsgeheimnis des Benutzers als Schlüssel, abgeschnitten auf die ersten 8 Bytes). Die Gesamtnutzlast beträgt 95 Bytes, die einfach verkettet und dann mit ASCII85 als QR-Code-Inhalt kodiert werden.

Das Venue-Gerät am Veranstaltungsort wird die empfangenen verschlüsselten Daten zunächst aus dem empfangenen QR-Code extrahieren. Danach prüft das Venue-Gerät vorzugsweise noch die Aktualität des im Code enthaltenen Check-in Zeitstempels 404 und verwirft den Code, falls der Zeitstempel älter als ein maximales Alter, z.B. 30 Sekunden, ist.

Dann kann das Venue-Gerät folgenden Datensatz z.B. mittels Verschlüsselung konkatenierter Werte (| | als Konkatenationsoperator) erstellen: AsymEnc_priv_Venue-Key(keyld | | ePubKey | | verificationTag | | encData | | venuePublicKey). Hierdurch wird der Wert encCheckInData erzeugt. Danach lädt das Nutzergerät die folgenden Daten zum Server hoch: Trace-ID, Venue-Gerät-ID, Zeitstempel, encCheckInData. Der Server speichert z.B. zumindest die Trace-ID, die Venue-ID ("locationID" bzw. "scannerID"), Check-in Zeit, die verschlüsselten Daten encCheckINData, den mac Wert, iv und den ephPublicKey.

In einer alternativen Ausführungsform ohne Venue-Gerät stellt der Venue-Betreiber nur ein Träger-Objekt bereit, z.B. einen Tischaufsteller mit QR-Code, die die Venue-ID des Venues kodiert. Der Benutzer scannt mit seinem Smartphone den QR-Code, der eine URL enthält. Die URL enthält eine Venue-ID, wobei das Einlesen der URL die App 218 dazu veranlasst, die Informationen des Venues vom Server 202 abzurufen, sowie optionale Venue-Zusatzdaten. Die App 218 kann diese URL als universal URL (deeplink) registrieren und die Venue-ID und den venuePublicKey über diesen Deeplink vom Server beziehen. Gemäß einer anderen Ausführungsform sind diese Daten bereits im QR-Code kodiert. Die App 218 generiert dann alle Informationen des QR-Codes wie oben beschrieben und verarbeitet diese wie das oben beschriebene Venue-Gerät (QR-Code-Daten mit venuePublicKey 512 neu verschlüsseln und die Trace-ID, Venue-ID, Eincheckzeit, verschlüsselte Daten, mac, iv und den ephemeren öffentlichen Schlüssel auf den Server hochladen).

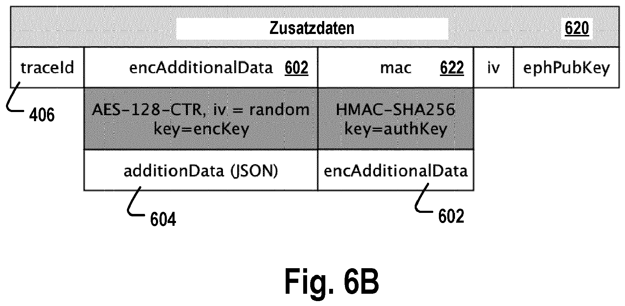

**Figuren 6A****,** **6B** zeigen Beispiele für Venue-Zusatzdaten 600, 620. Je nach Ausführungsform können die Venue-Zusatzdaten nur lokal auf den Venue-Geräten und/oder auf dem Server verknüpft mit den Venue-IDs gespeichert sein. Bei den Zusatzdaten handelt es sich um Daten, die sich auf den Nutzer und/oder dessen Umfeld beim Vorgang des Check-ins beziehen.

Beispielsweise können die Zusatzdaten weitere Angaben beinhalten, die der Betreiber des Venue benötigt, um dem Nutzer während seines Aufenthalts im Venue automatisch bestimmte Dienste bereitzustellen und/oder den Besuch in einer Venue-bezogenen Verwaltungssoftware zu dokumentieren und/oder zu organisieren. Beispielsweise können die Zusatzdaten eine Tischnummer beinhalten und den Tisch bezeichnen, an welchem sich ein Gast niederlässt. Die Zusatzdaten können sich auf eine Serviceleistung oder ein Fahrgeschäft beziehen, das der Besucher über eine App seines Nutzergerätes gewählt hat und/oder auf Daten, die zum Beispiel zur Anbahnung eines automatischen Zahlprozesses dienen. Beispielsweise kann während oder nach einem erfolgreichen Check-in das Venue-Gerät eine Anfrage an das Nutzergerät senden, und dadurch das Nutzergerät veranlassen, dem Nutzer über eine grafische Benutzeroberfläche zur Eingabe weiterer Zusatzdaten aufzufordern. Der Nutzer gibt also die für den Venue relevanten Daten (Tischnummer, gebuchtes Fahrgeschäft, Zusatz- oder Serviceleistungen, Alter des Nutzers etc.) ein, das Nutzergerät erzeugt daraufhin ein Datenobjekt (zum Beispiel im JSON Format), dass die vom Nutzer eingegebenen Daten beinhaltet, und sendet dieses an das Venue-Gerät zurück. Beispielsweise können die Anwendungen 218 auf dem Nutzergerät und die Anwendung 230 auf dem Venue Gerät als interoperabel Anwendungen eines verteilten Software-Systems zur datenschutzkonformen und sicheren Nachverfolgung von Besuchern implementiert sein.

Vorzugsweise werden die Venue-Zusatzdaten 604 in verschlüsselter Form 602 lokal und/oder auf dem Server verknüpft mit einer Trace-ID 406 des Check-im Vorgangs, in dessen Kontext die Zusatzdaten erfasst wurden, gespeichert. Beispielsweise kann ein Venuespezifischer Verschlüsselungsschlüssel 512 zur Verschlüsselung verwendet werden, sodass der Server nicht oder nur nach Zustimmung des Venue-Betreibers auf die Daten zugreifen kann.

Die Speicherung der Venue-Zusatzdaten kann vorteilhaft sein, da sich auf Basis zum Beispiel der Tischnummer oder des gewählten Fahrgeschäfts eines Vergnügungsparks der Aufenthaltsort des Besuchers noch genauer rekonstruieren lässt.

Zusätzlich oder alternativ dazu kann das Nutzergerät im Zuge eines Check-in Vorgangs auch Nutzer-Zusatzdaten erzeugen, welche mit dem UserDataKey (also dem nutzerspezifischen (öffentlichen asymmetrischen oder symmetrischen) Verschlüsselungsschlüssel dieses Nutzers und/oder mit dem Entitätenschlüssel verschlüsselt sind. Bei diesen Zusatzdaten handelt es sich um Daten, die ebenfalls im Kontext des Check-in Vorgangs stehen und welche das nachträgliche Tracing dieses Nutzers und/oder dessen Begleit- oder Kontaktpersonen erleichtern kann. Beispielsweise kann die App 218 dazu ausgebildet sein, den Nutzer beim Check-in (oder davor) aufzufordern, zu spezifizieren, mit welcher Begleitperson der Venue betreten wird und/oder welche sonstigen sich im Venue befindlichen Personen besucht werden sollen. Falls also ein Besucher eines Krankenhauses in Begleitung von Kindern erscheint und/oder einen bestimmten Patienten besucht, können diese Angaben zu den Kindern oder dem Patienten in Form der Nutzer-Zusatzdaten beim Check-in berücksichtigt und als Teil des Check-in Datensatzes auf dem Server gespeichert werden. Somit können auch Personen, die als Begleitpersonen, Besuchte oder in anderer Funktion Kontakt mit der zu tracenden Person hatten, leichter identifiziert werden auch dann, wenn diese Personen sich nicht gegenüber dem Server registriert haben.

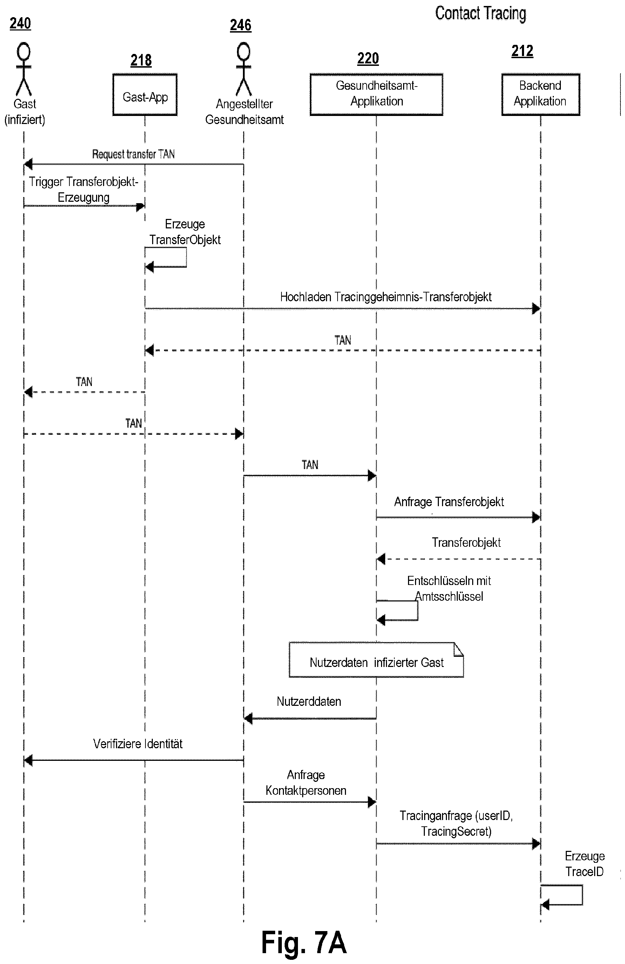

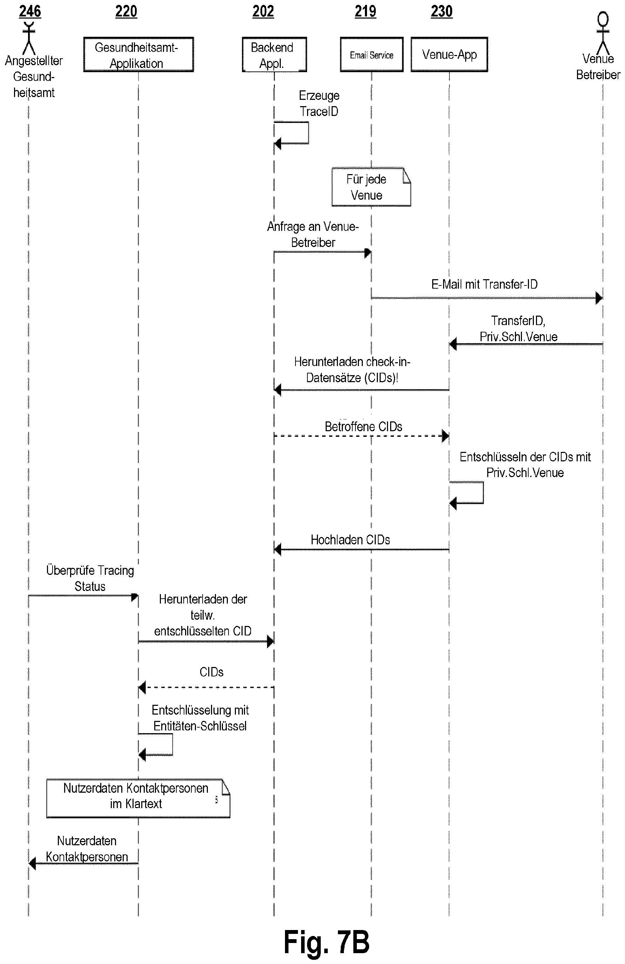

**Figur 7** zeigt ein UML-Diagramm eines Beispiels für das Tracing-Verfahren. Die von den gestrichelten Linien repräsentierten Personen bzw. Systemkomponenten entsprechen den in Figur 2 gezeigten Komponenten. Das Verfahren zum Tracing eines Nutzers integriert gemäß Ausführungsformen den gesamten Prozess vom ersten Kontakt eines infizierten Gastes mit dem Gesundheitsamt an bis zur Offenlegung der Kontaktdaten möglicher Kontaktpersonen gegenüber dem Amt. Um die Kontaktpersonen ausfindig zu machen, benötigt das Gesundheitsamt die TAN einer infizierten Person.

Die hier im Hinblick auf ein Gesundheitsamt und einen Pandemiefall beschriebenen Beispiele gelten analog auch für andere Typen von Entitäten und Notfälle.

Figur 7 illustriert den Fall, dass ein Nutzer (Gast), der in der Vergangenheit verschiedene Venues besucht hat, ein Testergebnis erhalten hat, wonach der Nutzer mit einer ansteckenden Krankheit (z.B. Covid 19) infiziert ist oder zumindest war. Dieses Testergebnis teilt der Nutzer, der Arzt oder das Labor der Gesundheitsbehörde mit. Bei einer Ansteckung werden die infizierten Gäste von der örtlichen Gesundheitsbehörde kontaktiert. Bei der Kontaktaufnahme wird der infizierte Gast gefragt, ob er das Tracing-System gemäß einer hier beschriebenen Ausführungsform der Erfindung benutzt, und er wird gebeten, seine persönlichen Daten und seine Kontakthistorie zur automatischen Kontaktverfolgung offenzulegen.

Der besagte infizierte Gast startet den Ablauf des Tracings, indem er ein Datenübertragungsobjekt (Tracinggeheimnis-Transferobjekt-) hochlädt, das verschiedene Geheimnisse enthält. Die App 218 erstellt das Tracinggeheimnis-Transferobjekt für den Austausch z.B. wie folgt:
- Serialisieren von userID, userDataKey und userTracingSecret in einem JSON Objekt;
- asymmetrische Verschlüsselung des serialisierten JSON Objekts mit dem aktuellen für diesen Tag gültigen Entitätenschlüssel dailyPublicKey durch das Nutzergerät
- die resultierenden verschlüsselten Daten, und optional auch iv, mac und ephPublicKey zum Server 202 hochladen und als Tracinggeheimnis-Transferobjekt dieses Nutzers auf dem Server speichern.
- Der Server wird eine TAN erzeugen, verknüpft mit dem besagten Tracinggeheimnis-Transferobjekt auf dem Server speichern und die TAN an das Nutzergerät zurückgeben. Beispielsweise kann der Server die TAN an das Nutzergerät des Nutzers zurückgeben, wo es auf dem Display des Geräts angezeigt wird, und der Nutzer kann die Tan telefonisch dem Gesundheitsamt mitteilen. Erst mit der TAN kann die Gesundheitsbehörde die Daten auf dem Server zur Kontaktverfolgung nutzen.

Nach Kontaktaufnahme mit dem infizierten Gast gibt der Mitarbeiter des Gesundheitsamtes die TAN in seiner Frontend-Applikation des Gesundheitsamtes ein. Der Mitarbeiter ist angemeldet z.B. unter Verwendung seines während des Registrierungsprozesses erzeugten Zertifikats, das die Identität des Benutzers verifiziert.

**Bezug des Entitäten-Schlüssels:** Damit der Prozess beginnen kann, benötigt der Mitarbeiter der Gesundheitsabteilung Zugang zu allen relevanten DailyKeypairs des Gesundheitsamts. Daher holt er sich das verschlüsselte, täglich neu erstellte asymmetrische Schlüsselpaar des Gesundheitsamts (HealthDepartmentEncryptionKeyPair), das er mit seinem persönlichen privaten Encryption-Key entschlüsseln kann. Mit dem lokal entschlüsselten healthDepartmentEncryptionKeyPair kann er die EncryptedDailyKeypairs abrufen und lesen.

In Ausführungsformen in welchen nur ein statisches, über eine lange Zeit gültiges asymmetrisches Schlüsselpaar für die Entität erstellt wird, kann der Bezug der Entitäten-Schlüssel auch einfacher sein und ein Lesen des privaten Entitäten-schlüssels umfassen.

### Abrufen der persönlichen Daten des infizierten Gastes:

Nachdem der angemeldete Mitarbeiter z.B. telefonisch oder per Mail oder Briefpost die TAN von dem Nutzer erfahren hat, sendet der Mitarbeiter des Amts eine Tracing-Anfrage an den Server, wobei die Anfrage die TAN beinhaltet. Der Server identifiziert das mit der TAN verknüpft gespeicherte Tracinggeheimnis-Transferobjekt und stellt es dem Mitarbeiter der Behörde zum Download bereit. Das Transferobjekt ist vorzugsweise mit dem aktuellen öffentlichen Schlüssel des Gesundheitsamts verschlüsselt und wird vom Mitarbeiter des Amts mit dem korrespondierenden privaten Schlüssel entschlüsselt.

Die dem Gesundheitsamt nun vorliegenden entschlüsselten Geheimnisse des Tracinggeheimnis-Transferobjekts enthalten die Userld und das UserTracingSecret des zu tracenden Nutzers, sodass das Amt (also ein Entitäten-Gerät, das dem Amt zugewiesen ist) die Kandidaten-Tracing-IDs dieses Gasts für z.B. die letzten 14 Tage (20.160 Kandidaten Trace-IDs) berechnen kann.

### Identifikation besuchter Venues und möglicher Kontaktpersonen

Das Gesundheitsamt sendet eine Anfrage an den Server, die die berechneten Kandidaten-Tracing-IDs beinhaltet. Der Server durchsucht seine Check-in Datensätze in seiner Datenbank und ermittelt alle Venue-IDs aller Check-in Datensätze, deren Trace-IDs identisch sind zu einer der in der Anfrage übermittelten Kandidaten-Trace-IDs. Die Kontaktdaten dieser ermittelten Venues werden vom Server ans Amt zurückgegeben.

Das Gesundheitsamt kann dann die Veranstaltungsorte (Venues bzw. die Venue-Geräte) auffordern, Zugang zu allen Check-in Datensätzen zu gewähren, die sich zur gleichen Zeit wie die infizierten Benutzer am Veranstaltungsort befanden. Die Veranstaltungsorte werden z.B. per Mail kontaktiert, um diese über das laufende Tracingverfahren zu informieren und um Freigabe der auf dieses Venue bezogenen Check-in Datensätze zu bitten.

Der Venue wird im Falle seiner Zustimmung alle Check-in Datensätze (oder alle Check-in Datensätze bis zu einem maximalen Alter, falls die Check-in Time im Klartext in den Check-in Datensätzen enthalten sein sollte) herunterladen, die von diesem Venue stammen, diese entschlüsseln und z.B. anhand der Zeitstempel 404 überprüfen, welche Gäste Kontaktpersonen sein könnten. Dann lädt das Venue-Gerät die Check-in Daten der möglichen Kontaktpersonen (deren sensible Daten wie die Ausweisdaten immer noch mit dem DailyPublicKey der Entität verschlüsselt sind) zum Server hoch, der diese Daten an das Gesundheitsamt weiterleitet. In einer alternativen Ausführungsform kann das Venue-Gerät die mit dem privaten Venue-Schlüssel entschlüsselten Daten auch direkt an das Entität-Gerät senden.

Dieser Umweg über den Venue ist nur in Ausführungsformen erforderlich, in welchen die Check-in Datensätze zusätzlich noch mit dem öffentlichen Venue-Schlüssel verschlüsselt sind und der Venue die Check-in Datensätze mit dem korrespondierenden Entschlüsselungsschlüssel wieder entschlüsseln muss damit die Entität sie verarbeiten kann. Falls die Check-in Daten nicht noch zusätzlich mit dem Venue-Schlüssel verschlüsselt sind, ist es auch möglich, dass die mit dem Entitätenschlüssel verschlüsselten Check-in Datensätze der möglichen Kontaktpersonen direkt vom Server an das Entitäten-Gerät übermittelt werden.

Nachdem das Entitäten-Gerät alle Check-in Datensätze der möglichen Kontaktpersonen im fraglichen Zeitraum erhalten hat und diese nicht (mehr) mit dem Venue-Schlüssel verschlüsselt sind, kann das Entitäten-Gerät diese Check-in Datensätze mit den entsprechenden DailyPrivateKeys der Entität entschlüsseln.

Nachdem der Entitäten-Rechner sowohl User-IDs als auch die UserDataKeys (also die zur Entschlüsselung der Nutzerdaten nötigen Entschlüsselungsschlüssel) möglicher Kontaktpersonen durch die Entschlüsselung erhalten hat, kann das Entitäten-Gerät vom Server die verschlüsselten Nutzerdatensätze dieser möglichen Kontaktpersonen anfordern und mit den jeweiligen nutzerspezifischen UserDataKeys entschlüsseln und Kontakt zu diesen Nutzern aufnehmen. Das Amt kann aber kein Tracing für diese Kontaktpersonen vornehmen, solange diese nicht deren Tracinggeheimnis-Transferobjekt dem Amt zur Verfügung stellen.

Die Gesundheitsbehörde kann nun zudem auch die Integrität des Check-ins mit dem Verifizierungsgeheimnis verifizieren.

Es ist für Ausführungsformen des elektronischen Systems somit unmöglich, die Benutzer ohne deren Zustimmung zu verfolgen, da die Check-in Datensätze verschlüsselt sind und die als Suchschlüssel verwendete Trace-IDs dieser Check-in Datensätze ohne Kenntnis weiterer Daten, z.B. des Tracing-Secrets, welches nutzerindividuell erzeugt wird und geschützt in den jeweiligen Nutzergeräten gespeichert ist, nicht korreliert werden können. Das TracingSecret wird aber nur im Infektionsfall und nur mit Zustimmung des Nutzers ans Amt übertragen. Benutzer, die nur Kontakt mit einem infizierten Benutzer hatten, werden ihr TracingSecret nicht preisgeben und daher nicht zurückverfolgt werden können.

Die persönlichen Daten des Benutzers sind zu jeder Zeit verschlüsselt und können nur von Personen gelesen werden, die Kenntnis vom UserDataKey haben. Der userDataKey kann nur vom Benutzer selbst oder in verschlüsselter Form von einem Venue-Gerät bezogen werden, das einen Check-in QR-Code des Nutzers gescannt hat. Alle Entschlüsselungsvorgänge werden vorzugsweise lokal durch die jeweiligen Systemkomponenten durchgeführt. Nach Ausführungsformen sind alle Daten, die auf dem Server und/oder den Systemkomponenten (Nutzergerät, Venue-Gerät, Entitäts-Gerät und/oder Server) gespeichert sind, in irgendeiner Weise vom Eigentümer der Daten signiert/gemac'ed (wurden also als Input einer MAC Funktion zur Berechnung eines mac Werts), so dass kein Mitarbeiter die Daten ändern kann.

## Patentansprüche

1. Computerimplementiertes Verfahren zum Tracing von Nutzern, die sich temporär in verschiedenen räumlichen Bereichen (Venues) aufhalten, für eine Entität (246), insbesondere für Notfallszenarien wie in einer Pandemie, wobei das Verfahren umfasst:
- anlässlich einer Registrierung (102) eines jeden der Nutzer: Speicherung von Nutzerdaten (306) des Nutzers auf einem Server (202), wobei die Nutzerdaten (314) von einem dem Nutzer zugeordneten Nutzergerät mit einem für den Nutzer individuellen User-Data-Key (310) verschlüsselt werden, wobei insbesondere eine User-ID (302) des Nutzers verknüpft mit den verschlüsselten Nutzerdaten (306) auf dem Server gespeichert wird; und Speicherung der U-ser-ID und eines Nutzer-individuellen, zur Entschlüsselung der verschlüsselten Nutzerdaten erforderlichen, User-Data-Entschlüsselungsschlüssels (310) in dem Nutzergerät, wobei vorzugsweise der User-Data-Key und/oder der User-Data-Entschlüsselungsschlüssel (310) geschützt gespeichert werden und dem Server nicht zugänglich sind;
- Im Zuge eines Check-ins (104) eines der Nutzer gegenüber einem der Venues, Erzeugung einer Trace-ID durch das Nutzergerät des einen Nutzers, wobei die Trace-ID repräsentativ ist für eine Kombination aus der User-ID des einen Nutzers und einer aktuellen Zeitinformation, und Speicherung der Trace-ID verknüpft mit der Venue-ID des einen Venue auf dem Server (202), wobei die Trace-ID als Zugriffsschlüssel gespeichert wird,
- Empfang (106) einer Mitteilung beinhaltend die User-ID eines der Nutzer, bezüglich welchem das Tracing durchgeführt werden soll;
- Erzeugung (108) von Kandidaten-Trace-IDs für den in der Mitteilung identifizierten Nutzer, wobei die Kandidaten-Trace-IDs mögliche zurückliegende Trace-IDs sind, die innerhalb eines vorgegebenen vergangenen Zeitraums liegen,
- Verwendung (110) der erzeugten Kandidaten-Trace-IDs als Suchkriterium durch den Server (202), um entsprechende auf dem Server für den vergangenen Zeitraum gespeicherte Trace-IDs aufzufinden, so dass die Venue-IDs (502) derjenigen Venues gegenüber welchen der zu tracende Nutzer während des vergangenen Zeitraums einen Check-in durchgeführt hat, ermittelt werden, und
- Ausgabe (112) der Venue-IDs der ermittelten Venues zur Identifizierung derjenigen Venues, die der zu tracende Nutzer während des vergangenen Zeitraums besucht hat, durch den Server (202).

2. Elektronisches System (200) mit Nutzergeräten (204), die jeweils einem Nutzer (240) zugeordnet sind und zumindest einem Server (202) zum Tracing von Nutzern, die sich temporär in verschiedenen räumlichen Bereichen (Venues) aufhalten, für eine Entität (246), wobei das elektronische System zur Durchführung der folgenden Schritte konfiguriert ist:
- anlässlich einer Registrierung (102): Speicherung von Nutzerdaten (300) der Nutzer auf dem Server (202), wobei die Nutzerdaten mit einem für den jeweiligen Nutzer individuellen User-Data-Key (310) verschlüsselt sind, wobei insbesondere eine User-ID (302) des betreffenden Nutzers verknüpft mit den verschlüsselten Nutzerdaten (306) auf dem Server gespeichert werden; und Speicherung der User-IDs und eines Nutzer-individuellen, zur Entschlüsselung der verschlüsselten Nutzerdaten erforderlichen, User-Data-Entschlüsselungsschlüssels (310) jeweils in demjenigen der Nutzergeräte, welches dem Nutzer zugeordnet ist, wobei vorzugsweise der User-Data-Key und/oder der User-Data-Entschlüsselungsschlüssel (310) geschützt in dem Nutzergerät des jeweiligen Nutzer gespeichert wird und dem Server nicht zugänglich ist;
- Im Zuge eines Check-ins (104) eines der Nutzer gegenüber einem der Venues, Erzeugung einer Trace-ID durch das Nutzergerät des einen Nutzers, wobei die Trace-ID repräsentativ ist für eine Kombination aus User-ID des einen Nutzers und einer aktuellen Zeitinformation, und Speicherung der Trace-ID verknüpft mit der Venue-ID des einen Venue auf dem Server (202), wobei die Trace-ID als Zugriffsschlüssel gespeichert wird,
- Empfang (106) einer Mitteilung beinhaltend die User-ID eines der Nutzer, bezüglich welchem das Tracing durchgeführt werden soll;
- Erzeugung (108) von Kandidaten-Trace-IDs für den in der Mitteilung identifizierten Nutzer, wobei die Kandidaten-Trace-IDs mögliche zurückliegende Trace-IDs sind, die innerhalb eines vorgegebenen vergangenen Zeitraums liegen,
- Verwendung (110) der erzeugten Kandidaten-Trace-IDs als Suchkriterium durch den Server (202), um entsprechende auf dem Server für den vergangenen Zeitraum gespeicherte Trace-IDs aufzufinden, so dass die Venue-IDs derjenigen Venues gegenüber welchen der zu tracende Nutzer während des vergangenen Zeitraums einen Check-in durchgeführt hat, ermittelt werden, und
- Ausgabe (112) der Venue-IDs der ermittelten Venues zur Identifizierung derjenigen Venues, die der zu tracende Nutzer während des vergangenen Zeitraums besucht hat, durch den Server (202).

3. Elektronisches System nach Anspruch 2,
- wobei die im Zuge des Check-ins erzeugte Trace-ID mittels einer Ableitfunktion als Funktion der Kombination der zu repräsentierenden User-ID und der zu repräsentierenden aktuellen Zeitinformation beim Check-in berechnet wird; und
- wobei jeder der Kandidaten-Trace-IDs jeweils mittels der gleichen Ableitfunktion als Funktion der Kombination der in der Mitteilung enthaltenen User -ID und einer der Kandidaten-Zeitinformationen für einen möglichen in der Vergangenheit liegenden Check-in-Zeitpunkt, berechnet wird,
- wobei vorzugsweise die Ableitfunktion umfasst eine Rundung der aktuellen bzw. Kandidaten-Zeitinformation auf Zeitpunkte innerhalb vordefinierter Zeitintervalle, wobei die Zeitintervalle insbesondere Minuten oder Stunden oder Tage sind.

4. Elektronisches System nach Anspruch 3,
- Wobei das Nutzergerät dazu konfiguriert ist, im Zuge der Registrierung gegenüber dem Server einen Zufallswert (userTracingSecret, 410) zu erzeugen und zugriffsgeschützt ausschließlich im Nutzergerät zu speichern;
- wobei die Ableitfunktion die Trace-ID als Funktion des Zufallswerts (410) berechnet;
- wobei das Nutzergerät dazu konfiguriert ist, von dem Benutzer, dem es zugewiesen ist, eine Bestätigung zur Übermittlung des Zufallswerts einzuholen, und erst in Antwort auf die Bestätigung den Zufallswert an den Server oder an ein Entität-Gerät der Entität zu übermitteln um dem Server oder der Entität die Ableitung der Kandidaten-Trace-IDs zu ermöglichen.

5. Elektronisches System nach einem der vorigen Ansprüche 2-4,
- Wobei das Nutzergerät dazu konfiguriert ist, im Zuge der Registrierung gegenüber dem Server einen Zufallswert (412) zu erzeugen, im Folgenden als userVerificationSecret bezeichnet, und zugriffsgeschützt ausschließlich im Nutzergerät zu speichern;
- wobei das Nutzergerät dazu konfiguriert ist, im Zuge des Check-ins gegenüber dem Venue das userVerificationSecret zu verwenden, um mit diesem einen Verifikationsdatenwert (414) als Funktion der Trace-ID (406) und der verschlüsselten Ausweis-Daten (408) zu berechnen und verknüpft mit der Trace-ID auf dem Server zu speichern.

6. Elektronisches System nach einem der vorigen Ansprüche 2-5,
- wobei der Entität ein asymmetrisches kryptographisches Schlüsselpaar mit einem öffentlichen und einem privaten Entitätenschlüssel zugeordnet ist,
- wobei das Nutzergerät des Nutzers dazu konfiguriert ist, im Zuge der Registrierung die bereits mit dem Nutzerdaten-Schlüssel (310) verschlüsselten Nutzerdaten zusätzlich mit dem öffentlichen Entitätenschlüssel (332) zu verschlüsseln, und/oder
- wobei das Nutzergerät des Nutzers im Zuge des Check-ins dazu konfiguriert ist, den für die Entschlüsselung dieser verschlüsselten Nutzerdaten (306) erforderlichen Nutzerdaten-Entschlüsselungsschlüssel (310) durch den öffentlichen Entitätenschlüssel zusammen mit der User-ID des Nutzers zu verschlüsseln, um verschlüsselte Ausweis-Daten (408) zu erzeugen, und dazu konfiguriert ist, die verschlüsselten Ausweis-Daten (408, 506) im Zuge des Check-ins verknüpft mit der Venue-ID (502) des einen Venue auf dem Server (202) zu speichern.

7. Elektronisches System nach Anspruch 6, ferner umfassend zumindest ein Entitäts-Gerät (208), das der Entität zugeordnet ist,
- wobei das Entitäts-Gerät dazu konfiguriert ist, nacheinander mehrere asymmetrische kryptographische Schlüsselpaare zu erzeugen, die der Entität zugewiesen sind, wobei bei Erzeugung eines neuen Entitäten-Schlüsselpaares das bisher gültige invalidiert wird, wobei jedem der Entitäten-Schlüsselpaare eine Schlüsselversionsnummer zugewiesen ist,
- wobei das zumindest eine Entitäts-Gerät dazu konfiguriert ist, den öffentlichen Schlüssel des erzeugten und aktuell gültigen Schlüsselpaares an die Nutzergeräte zu übertragen;
- wobei die Nutzergeräte dazu konfiguriert sind, immer nur den jüngsten übertragenen Entitätenschlüssel (332) zur Erzeugung der mehrfach verschlüsselten Nutzerdaten und/oder zur Erzeugung der verschlüsselten Ausweis-Daten (408) zu verwenden.

8. Elektronisches System nach Anspruch 7, wobei das zumindest ein Entitäts-Gerät (208) eine Vielzahl von Entitätsgeräten umfasst,
- wobei dasjenige der Entitäts-Geräte, welches das aktuelle asymmetrische Entitäten-Schlüsselpaar erzeugt, den privaten Schlüssel des aktuell erzeugten asymmetrischen Entitäten-Schlüsselpaares in verschlüsselter Form an alle anderen der Entitäts-Geräte über ein Netzwerk zu senden,
- wobei jedes der Entitäts-Geräte dazu konfiguriert ist, eine Mitteilung eines Notfalls mit Bezug zu einem der Nutzer zu erzeugen und an den Server (202) zu senden und/oder dazu konfiguriert ist, mittels eines der bisher empfangenen privaten Entitätenschlüssel die verschlüsselten Nutzerdaten und/oder die verschlüsselten Ausweis-Daten (408) zu entschlüsseln.

9. Elektronisches System nach einem der vorigen Ansprüche 2-8, ferner umfassend mehrere Venue-Geräte (206), wobei jedem der Venues ein oder mehrere der Venue-Geräte zugewiesen sind.

10. Elektronisches System nach Anspruch 9, wobei der Nutzer den Check-in gegenüber einem der Venue-Geräte (206) durchführt, wobei das eine Venue-Gerät eine Kommunikationsschnittstelle zur direkten Kommunikation mit dem Nutzergerät eines eincheckenden Nutzers beinhaltet, wobei das Nutzergerät und das eine Venue-Gerät so konfiguriert sind, dass im Zuge des Check-ins folgendes erfolgt:
- Erzeugung des Check-in Datenpakets (400, 420, 430) durch das Nutzergerät, wobei das Check-in Datenpaket die Trace-ID (406) und vorzugweise auch die verschlüsselten Ausweisdaten (408) kodiert;
- Übertragung des Check-in Datenpakets vom Nutzergerät über die direkte Kommunikationsschnittstelle an das Venue-Gerät;
- Dekodieren des empfangenen Check-in Datenpakets durch das Venue-Gerät, um die Trace-ID und vorzugsweise auch die Ausweisdaten (408) des eincheckenden Nutzers zu erhalten und diese verknüpft mit der Venue-ID des Venue, dem das Venue-Gerät zugewiesen ist, auf dem Server (202) zu speichern;
- wobei es sich vorzugsweise bei der direkten Kommunikationsverbindung handelt um eine Nahfeld-Kommunikationsverbindung oder um eine optische Datenübertragung, wobei die optische Datenübertragung eine Erfassung eines graphischen Codes, der das Check-in Datenpaket kodiert und auf einer Anzeige des Nutzergeräts angezeigt wird, durch einen optischen Sensor des Venue-Geräts umfasst.

11. Elektronisches System nach einem der vorigen Ansprüche 2-10, ferner umfassend zumindest ein Trägerobjekt mit einem graphischen Venue-Code eines der Venues, wobei der Venue-Code eine Venue-ID kodiert, unter welcher dieser Venue bei dem Server (202) registriert ist, wobei der Nutzer den Check-in gegenüber diesem Venue vornimmt, wobei das Nutzergerät so konfiguriert ist, dass im Zuge des Check-ins folgendes erfolgt:
- Erfassung des graphischen Venue-Codes mit einer Kamera des Nutzergerätes;
- Dekodierung des Venue-Codes durch das Nutzergerät, um die Venue-ID des Venues zu erhalten;
- wobei die Übertragung der Trace-ID und vorzugsweise auch der Ausweisdaten (408) des eincheckenden Nutzers an den Server durch das Nutzergerät erfolgt, wobei hierbei auch die dekodierte Venue-ID verknüpft mit der Trace-ID auf dem Server (202) gespeichert wird;
- wobei es sich bei dem Venue-Code vorzugsweise um einen QR-Code oder Barcode handelt, der auf einem Aufdruck oder einer Digitalanzeige sichtbar ist, handelt und/oder wobei es sich bei dem Trägerobjekt vorzugsweise handelt um einen Tischaufsteller oder ein Türschild zu einem Fahrzeugwagen, Fahrzeugabteil, Gebäude oder Zimmer.

12. Elektronisches System nach einem der vorigen Ansprüche 2-11, wobei das elektronische System so konfiguriert ist, dass von dem Nutzergerät des einen der Nutzer oder von dem Venue-Gerät eine Check-out Zeit an den Server übertragen wird, wenn der Nutzer den Venue verlässt, wobei die Check-out Zeit der zuvor für diesen Nutzer empfangenen Trace-ID zugeordnet wird, so dass für den Nutzer der Aufenthaltszeitraum an dem Venue gespeichert wird.

13. Elektronisches System nach einem der vorigen Ansprüche 2-12, wobei das System konfiguriert ist zur:
- Erzeugung eines Tracinggeheimnis-Transferobjekts durch das Nutzergerät, wobei das Tracinggeheimnis-Transferobjekt zumindest die User-ID und den Nutzerdaten-Entschlüsselungsschlüssel des Nutzers, dem das Nutzergerät zugewiesen ist, enthält;
- Verschlüsselung des Tracinggeheimnis-Transferobjekts mit einem öffentlichen Schlüssel (332) der Entität durch das Nutzergerät, wobei es sich bei dem öffentlichen Schlüssel insbesondere um den jüngsten aus einer Reihe nacheinander erzeugter öffentlicher Entitätenschlüssel handelt;
- Übertragung des verschlüsselten Tracinggeheimnis-Transferobjekts über ein Netzwerk von dem Nutzergerät an den Server;
- In Antwort auf den Erhalt des verschlüsselten Tracinggeheimnis-Transferobjekt, Erzeugen einer TAN und Speicherung der TAN verknüpft mit dem verschlüsselten Tracinggeheimnis-Transferobjekt durch den Server, und Ausgabe der TAN an den Nutzer;
- Empfang der TAN von dem Nutzer durch ein der Entität zugewiesenes Entitäts-Gerät (208);
- Verwenden der TAN durch das Entitäts-Gerät, um in Interoperation mit dem Server die verschlüsselten Nutzerdaten des Nutzers zu empfangen und zu entschlüsseln.

14. Elektronisches System nach Anspruch 13, wobei das Verwenden der TAN umfasst:
- Senden einer ersten Nutzerdatenanfrage beinhaltend die TAN an den Server (202) durch das Entitätsgerät (208);
- Empfang einer ersten Nutzerdatenanfrage beinhaltend die TAN durch den Server (202) von dem Entitätsgerät;
- In Antwort auf den Empfang der Nutzerdatenanfrage, senden des verschlüsselten Tracinggeheimnis-Transferobjekts, das mit der TAN verknüpft gespeichert ist, von dem Server an das Entitäts-Gerät;
- Entschlüsseln des Tracinggeheimnis-Transferobjekts mit dem privaten Entitätenschlüssel, der zu dem öffentlichen Schlüssel (332) der Entität korrespondiert, um die User-ID und den zur Entschlüsselung der verschlüsselten Nutzerdaten des Nutzers erforderlichen User-Data-Entschlüsselungsschlüssel (310) zu erhalten, durch das Entitäten-Gerät; und
- Verwenden der entschlüsselten User-ID, um eine zweite Nutzerdatenanfrage beinhaltend die User-ID zu erzeugen und vom Entitäten-Gerät an den Server zu senden; und
- Empfang der verschlüsselten Nutzerdaten des Nutzers vom Server durch das Entitäten-Gerät in Antwort auf die zweite Anfrage; und
- Entschlüsselung der verschlüsselten Nutzerdaten mittels des in dem Tracing-geheimnis-Transferobjekt enthaltenen Nutzerdaten-Entschlüsselungsschlüssels durch das Entitäten-Gerät.

15. Elektronisches System nach Anspruch 13 oder 14,
- wobei das Tracinggeheimnis-Transferobjekt zusätzlich ein im Zuge der Registrierung des gegenüber dem Server erzeugten und zugriffsgeschützt im Nutzergerät gespeicherten Zufallswert (userTracingSecret, 410) enthält;
- wobei der Server oder ein Entität-Gerät der Entität dazu konfiguriert ist, die Kandidaten-Trace-IDs als Funktion des Zufallswerts, von Zeitpunkten innerhalb des zurückliegenden Zeitraums und der User-ID zu berechnen.

16. Elektronisches System nach einem der Ansprüche 13-15,
- wobei das Tracinggeheimnis-Transferobjekt zusätzlich ein im Zuge der Registrierung des gegenüber dem Server erzeugten und zugriffsgeschützt ausschließlich im Nutzergerät gespeicherten Zufallswert (412) enthält, wobei der Zufallswert im Folgenden als userVerificationSecret bezeichnet wird;
- wobei der Server dazu konfiguriert ist:
o die auf dem Server gespeicherten Trace-IDs, die identisch zu einer der Kandidaten-Trace-IDs sind, zu ermitteln;
o das im Tracinggeheimnis-Transferobjekt enthaltene userVerificationSecret zu verwenden, um mit diesem einen Verifikationsdatenwert für jede der ermittelten Trace-IDs als Funktion der Trace-ID (406) und der mit diesen ermittelten Trace-IDs verknüpft gespeicherten verschlüsselten Ausweis-Daten (408) zu berechnen;
o den für eine jede der ermittelten Trace-IDs jeweils berechneten Verifikationsdatenwert mit einem Verifikationsdatenwert (414) zu vergleichen, der im Zuge des Check-ins verknüpft mit der ermittelten Trace-ID gespeichert wurde;
o ein Ergebnis dieses Vergleichs an das zumindest eine Entitäten-Gerät zu kommunizieren; und
o wobei das zumindest eine Entitäten-Gerät dazu konfiguriert ist, im Falle des Ergebnisses, dass der Vergleich eine fehlende Übereinstimmung der Verifikationsdatenwerte ergibt, die Zuweisung der Trace-ID an den Nutzer als manipuliert zu behandeln.

17. Elektronisches System nach einem der vorigen Ansprüche 2-16, wobei das elektronische System so konfiguriert ist, dass diejenigen Nutzer ermittelt werden, die ebenfalls einen oder mehrere der ermittelten Venues während des vergangenen Zeitraums und während einer Aufenthaltsdauer des Nutzers besucht haben, wobei die Ermittlung dieser Nutzer umfasst eine Ermittlung derjenigen der Trace-IDs und mit diesen verknüpft gespeicherten Check-in Datensätzen (400, 420, 430, 500, 520, 530), die von den ermittelten Venues während des vergangenen Zeitraums zu dem Server übertragen wurden.

18. Elektronisches System nach einem der vorigen Ansprüche 2-7, wobei die Trace-IDs die User-ID nicht im Klartext enthalten und wobei die Trace-IDs so erzeugt werden, dass allein auf Basis einer Analyse der auf dem Server gespeicherten Trace-IDs nicht festgestellt werden kann, ob zwei Trace-IDs die gleiche oder unterschiedliche User-IDs repräsentieren.

19. Digitales Speichermedium mit von einem Prozessor eines Datenverarbeitungsgeräts ausführbaren Programminstruktionen zur Durchführung derjenigen Schritte zu deren Durchführung des elektronische System nach einem der vorigen Ansprüche 2-18 konfiguriert ist, welche das Datenverarbeitungsgerät betreffen, wobei das Datenerarbeitungsgerät das Nutzergerät oder der Server (202) oder ein Venue-Gerät oder ein Entitäten-Gerät ist.
